(19) **Europäisches Patentamt**

**European Patent Office**

**Office européen des brevets**

(11) **EP 1 219 259 B1**

(12) **EUROPEAN PATENT SPECIFICATION**

(45) Date of publication and mention
of the grant of the patent:
**16.07.2003 Bulletin 2003/29**

(51) Int Cl.⁷: **A61B 19/00**

(21) Application number: **02004032.5**

(22) Date of filing: **22.04.1994**

(54) **System for locating relative positions of objects**

Anordnung zur Bestimmung der gegenseitigen Lage von Körpern

Système de détermination de la position relative d'objets

(84) Designated Contracting States:
**DE FR GB IT SE**

(30) Priority: **22.04.1993 US 52042**
**22.04.1993 US 52045**

(43) Date of publication of application:
**03.07.2002 Bulletin 2002/27**

(62) Document number(s) of the earlier application(s) in
accordance with Art. 76 EPC:
**94915394.4 / 0 700 269**

(73) Proprietor: **Image Guided Technologies, Inc.**
**Boulder, CO 80301 (US)**

(72) Inventor: **Schulz, Waldean A.**
**Boulder, CO 80301 (US)**

(74) Representative: **Röthinger, Rainer**
**c/o Wuesthoff & Wuesthoff**
**Patent- und Rechtsanwälte**
**Schweigerstrasse 2**
**81541 München (DE)**

(56) References cited:
**DE-A- 4 032 207**     **US-A- 4 722 056**
**US-A- 4 793 355**     **US-A- 4 896 673**
**US-A- 5 197 476**     **US-A- 5 309 913**

## Description

## FIELD OF THE INVENTION

This invention relates to locating the position of one object relative to another object in a three-dimensional space. It more particularly refers to a system for locating and displaying the position and the orientation of a first moving object relative to a second moving object in three dimensional space.

## Description of Prior Art

Computed tomography (CT), magnetic resonance imaging (MRI), and other methods provide important detailed images of the internals of human medical patients which are useful for diagnostic purposes. Often, these diagnostic tools, which are used to determine and display images of the inside of patients' bodies, were used, and the images they create were taken at times other than during actual surgical work on the patients, that is before and/or sometimes after surgery. Specifically, it is usual for CT or MRI scans to be taken before the surgeon starts his work. They are diagnostic tools, not tools for following an operation in progress. These prior scans are then commonly used to plan the surgery or at least to assist the surgeon in deciding what surgical course of action should be initiated and then followed. Sometimes, they are also used after surgery to determine and evaluate the results of the surgical procedure.

If surgery is in areas of the body which are not readily visible to the surgeon, such as inside the cranium within the recesses of the brain, it is difficult, if not impossible to follow the course of the surgery while it is going on. It is presently impossible to conduct surgery while simultaneously taking an MRI. Therefore, the surgeon is always working with previously taken images of the internal structures of the patient.

While working with these previously taken images during surgery, there often is no obvious, clear-cut relationship between points of interest in the diagnostic images and the corresponding points on the actual patient. While anomalous tissue may be obviously distinct from normal healthy tissue in the images, the difference may not be so visible in the patient on the operating table. Furthermore, in intracranial surgery, the region of interest may not always be accessible to direct view. Thus there exists a need for apparatus and systems to help a surgeon relate locations in the diagnostic images to the corresponding locations in the actual anatomy of the patient, and vice versa.

The related prior art includes references which disclose subject matter which describe means to accomplish objects which are similar to those of the present invention as a whole. These references include publications describing the correlation of previously taken internal medical images of a patient, which are usually three-dimensional, with the corresponding actual, present time physical locations on and in the patient in the operating room during surgery. U.S. Patent 4,791,934 describes a semi-automated system which makes this correlation, but note should be taken that the system described in this patent also requires additional radiographic imaging to be accomplished in the operating room at the time of surgery, and it then also requires that these present time images be correlated into the coordinate systems of the previously taken diagnostic images so that they can be related to the live patient.

Furthermore, the system of this '934 patent uses a computer-driven robot arm to position a surgical tool in relation to these images. It does not measure and define the present time location and orientation of an input probe (a surgical tool) positioned interactively by the surgeon, and superimpose such present time data on a previously taken image.

There have been other attempts to solve the three-dimensional localization problem specifically for stereotactic surgery. One class of solutions has been the use of a variety of mechanical frames, holders, or protractors for surgery (usually intracranial surgery). For examples see U.S. Patents 4,931,056, 4,875,478, 4,841,967, 4,809,694, 4,805,615, 4,723,544, 4,706,665, and 4,651,732, 4,638,798. Generally these patents disclose systems which are intended to reproduce angles derived from the analysis of internal images, and most require rigidly screwing a frame to the skull. In any case, these methods are all inconvenient, time-consuming, and prone to human error.

A more interactive method is disclosed in United States patent 4,750,487. This patent discloses the use of present time fluoroscopy in the operating room to help guide surgical tools. It will be abundantly clear that subjecting a patient to fluoroscopy is undesirable at any time. It may impose even greater risks during surgery.

More relevant prior art are the publications which describe systems which have been built specifically for stereotactic surgery. The following reference is illustrative and pertinent:

> David W. Roberts, M.D., et al; "A Frameless Stereotaxic Integration of Computerized Tomographic Imaging and the Operating Microscope", <u>J. Neurosurgery 65</u>, Oct. 1986.

This reference reports on the use of a three-dimensional digitizer to track the position and orientation of the field of view of a surgical microscope and means to superimpose, on the field of view in the microscope, the corresponding internal planar slice of a previously obtained computed tomographic (CT) image. Such a system was a great step forward in this art. It was, however, not without attendant disadvantages.

In the first place, the system described by Roberts et al. used the transmission of sound impulses to communicate the location and orientation of the field of the surgical microscope. The use of sound is necessarily based on the determination of transmission distances as a function of the speed of sound, and the differentiation of small distance differences. It is well known that the speed of sound varies to a substantial extent as a function of the temperature in the medium through which the sound travels. With modern air conditioning and heating, there are many different thermoclines and air currents present in an operating room, which are of little concern to a patient or to the doctors, but which can materially effect the accurate measurement of precise distances as a function of the speed of sound transmission. Therefore very accurate compensation factors must be applied in order to get accurate information on the exact location and orientation of the operating microscope. It will be appreciated that small errors in this field can have very serious consequences to the patient.

It must be remembered that the purpose of measuring distances from the sound emitters to the receivers is to determine precisely where the point of a surgical probe, or other device, is. The point may move only a very short distance, millimeters or even less, and so the position of the sound emitter on the probe my change by only a very small amount. This mensuration system must be able to detect such small changes in position and orientation of the sound emitters, and to translate these small changes into meaningful changes in the defined position and orientation of the point of the probe. Thus the accuracy of measuring and knowing the speed of sound in the medium of the operating room is critical to locating the point of the probe with any accuracy and cross correlating this location with a previously taken image which corresponds to the location of the probe tip. It should therefore be clear that the major disadvantage for this reported sonic system is the inherent inaccuracy and instability of the sonic mensuration apparatus.

In addition to the difficulty of making accurate and precise distance measurements using sound transmission, there was another difficulty and uncertainty which was inherent in the Roberts et al. system regardless of its use of sound sensing means or not. The Roberts et al. system relied on the position of the patient, at least so much of the patient as corresponded to the area being operated on-that is for example the head, being absolutely fixed. That system was not capable of determining changes in the location of the patient housing the operating microscope, but tracked only the location of the microscope independent of the patient. The location and orientation of the patient (the head of the patient) was determined at the start of the operation and the previously taken CT scan related to that specific position and orientation. If this position and/or orientation of the patient was changed, intentionally or even inadvertently, during the entire operation, the system had to be re-correlated. That is, the operation had to be stopped and the previously taken CT scan had to be correlated with the new position and/or orientation of the patient's head.

Clearly this was a major disadvantage of the Roberts et al. system. Not only does it require that the system be re-correlated at the time that the surgeon intentionally moved the patient's head, but it was not capable of taking into consideration inadvertent movements of the patient's head during the operation.

The present invention does not include the taking of suitable images of the internals of a patient before the operation. It starts from the point at which these images have already been taken and are found to be acceptable by the surgeon. This invention therefore does not comprise the imaging apparatus used to generate the internal three-dimensional image or model of the internals of the patient or other object. However, this invention does use these previous imaging data and inputs this information into the instant system. The system of this invention does not include the means of taking these images, but it does include the images themselves, preferably in electronic form.

It is contemplated that such known imaging devices might be ultra-sound, computed tomography (CT) or magnetic resonance imaging (MRI). It is also contemplated that such imaging device might be one which has as yet not been developed. The important limiting factor in the sense of the imager is that the data generated by that imager must be available in an electronic digital format, or is readily convertible into such format. The digital data may be derived directly from such an imager and transmitted to the instant system over a conventional communication network or through magnetic tape or disk media.

There is another area of prior art references which may be applicable to the patentability of the instant invention. This prior art is related specifically to localizing devices, which measure the relative positions of a manually maneuvered probe and another object, but not necessarily applied to "seeing" the present time position of the probe internal to a patient. Previous methods and devices have been utilized to sense the position of an object in three-dimensional space. These known techniques employ various methods of mensuration.

Numerous three-dimensional mensuration methods project a thin beam or a plane of light onto an object and optically sense where the light intersects the object. Examples of several United States patents which disclose such simple distance range-finding devices using this general approach are: U.S. patents 4,660,970, 4,701,049, 4,705,395, 4,709,156, 4,733,969, 4,743,770, 4,753,528, 4,761,072, 4,764,016, 4,782,239, and 4,825,091. Examples of United States patents which disclose using the plane of light to sense an object's shape include: U.S. patents 4,821,200, 4,701,047, 4,705,401, 4,737,032, 4,745,290, 4,794,262, 4,821,200, 4,743,771, and 4,822,163. In the latter, the accuracy of determining the location and orientation of the surface sample points is limited by the typically low resolution of two-dimensional sensors which have usually been employed (currently the accuracy of these devices is about 1 part in 512 for a solid state video camera). Furthermore, these devices are not presently capable of detecting the location and orientation of the tip of a probe whereby the tip of the probe identified is coincident with specific points, and identifying the location of such probe tip is synonymous with identifying such specific point. Additionally, optical systems are traditionally limited by line-of-sight considerations. If you cannot see the point in question, light cannot be directly impinged on that point or projected from it. Because of these inherent limitations, these known devices have been generally useless for locating a point within a recess, which is necessary for intracranial surgery.

The internal imaging devices themselves (such as computed tomography, magnetic resonance imaging, or ultrasonic imaging) are unsuited for tracking the spatial location and orientation of a manually held probe during an operation, even though they are unencumbered by line-of-sight restrictions. Thus, these systems are not capable of being used to previously record an image, or a set of images, of the internals of a patient, and also to image these same internals in present time during an operation.

Other prior art methods and apparatus are known which track the position of one or more specific moveable points in three-dimensional space. In these known techniques, the moveable points are generally represented by small radiating emitters which move relative to fixed position sensors. Some methods interchange the roles of the emitters and sensors. The typical forms of radiation are light (U.S. Patent 4,836,778 for example), sound (U.S. Patent 3,821,469), and magnetic fields (U.S. Patent 3,983,474). Other methods include mechanical arms or cables (U.S. Patent 4,779,212). Some electro-optical approaches use a pair of video cameras plus a computer to calculate the position of homologous points in a pair of stereographic video images (for example, U.S. Patents 4,836,778 or 4,829,373). The points of interest may be passive reflectors or flashing light emitters. The use of light emitters tend to simplify finding, distinguishing, and calculating the location and orientation of the points.

Probes with a pointing tip and sonic localizing emitters on them have been publicly marketed for several years. The instant invention is also concerned with determining the location and orientation of a stylus, but it is an improvement over the known devices in that it employs tiny light emitters, in place of the known sound emitters. Further, as will become apparent, the method used to sense the positions of these light emitters is different from what has been used in connection with sound.

Additional prior art related to the instant invention is found in these references:

Fuchs, H.; Duran, J.; Johnson, B.; "Acquisition and Modeling of Human Body Form Data", Proc. SPIE, v 166, (1978), p 94-102.

Mesqui, F.; Kaeser, F.; Fischer, P.; "Real-time, Noninvasive Recording and 3-d Display of the Functional Movements of an Arbitrary Mandible Point", SPIE Biostereometrics 602, 1985, p 77-84.

Yamashita, Y.; Suzuki, N.; Oshima, M.; "Three-Dimensional Stereometric Measurement System Using Optical Scanners, Cylindrical Lenses, and Line Sensors, Proc. SPIE, v. 361, 1983, p. 67-73.

The paper by Fuchs, et al, (1978) best describes a method used to track a surgical probe in three-dimensional space. This method could possibly be used in conjunction with and to supplement the practice of the instant invention. It is based on using three or more one-dimensional sensors, each consisting of a cylindrical lens and a linear array of radiation detectors, such as a charge-coupled semiconductor device (CCD) or a differential-voltage position sensitive detector (PSD). The sensors determine intersecting planes which all correspond to a single point radiating light emitter. Calculation of the point of intersection of the planes gives the location of the emitter. The calculation is based on the locations, orientations, and other details concerning the one-dimensional sensors and is a straightforward application of analytic geometry. This photo-optical method, however, has not been previously used for the purpose of the present invention. In that sense, it is possible that the instant system could be considered to be a new and unobvious use of an existing system.

A reference display system for superimposing a tomographic image onto the focal plane of an operating microscope is known from US 4,722,056. The system comprises a computer, which is programmed to reformat previously stored tomographic image data to present the same at a determinable plane, and an operating microscope positioned in the operative location relative to a patient, the focal plane of the microscope establishing said determinable plane. The system further comprises means for determining the spatial relationships of the imaging system, the patient, and the focal plane of the microscope with respect to one another. In order to allow for a correct superimposing, at least three fiducials are attached to the head of the patient. The fiducials are composed of a material that is physically detectable during the generation of the image data as well as visually to the microscope.

In order to establish the spatial relationship between the microscope and the fiducials, as well as to track any subsequent movement of the microscope, radiation emitters are located on the microscope and corresponding radiation sensors are arranged in a fixed position in the operating room. During establishment of the spatial relationship between the microscope and the fiducials, as well as during the tracking of any subsequent movement of the microscope relative to the fiducials, i.

e., relative to the patient's head, the patient's head is rigidly clamped to the operating table.

There still remains a need for a complete system (apparatus and method) which provides fast, accurate, safe, and convenient mensuration of the three-dimensional position and orientation of a manually operated probe relative to a moveable object of interest. This system must also visually relate, in the same coordinate system, the relative position and orientation of the probe, even a portion of the probe, which is out of line of sight, to an image of a previously-generated three-dimensional model of the object.

## Objects and Summary of the Invention

one objective of the present invention is to provide means for accurate three-dimensional mensuration of the relative position and orientation of a moveable member with respect to a moveable object.

Another object of this invention is to provide accurate visual relationship between two objects which are each moveable with respect to each other as well as with respect to the coordinate system in which these movable objects reside.

A further object of this invention is to provide accurate spacial relationships between a moving probe and a moving surgical patient during an operation in an operating room, wherein the probe and the patient are moving relative to each other as well as relative to a fixed location and orientation of the mensuration apparatus.

A still further object of this invention is to provide an electro-optical mensuration system which is inexpensive, easy to use, reliable, and portable, and which employs a manually positioned probe, or other instrument, at least part of which is not within a line of sight of the surgeon, and which further employs a means of measuring the otherwise "invisible" position and orientation of the probe tip.

Another object of this invention is to provide a simple, non-invasive system for establishing a correspondence between a presently existing coordinate system containing a movable object and a previously obtained coordinate system containing a three-dimensional computer model of that object, where the previously obtained computer model is also of this same system.

Another object of this invention is to relate a measured location on the outside, or inside, of an object to its corresponding location in a previously generated computer model of that object by establishing correspondence between the coordinate systems of the object and the model.

Another object of this invention is to display a cut-away view or a cross-sectional slice of a previously generated computer model of a planar cross-section of a geometric model, where the slice approximately intersects the location in the model corresponding to a location measured in present time, and to superimpose a marker on the displayed slice to indicate the location on the slice corresponding to the measured location.

Another object of this invention is to assist an operating surgeon locate subcutaneous diseased tissue while avoiding healthy critical structures, especially in cranial neurosurgery.

Additional objects, advantages, and novel features of the invention shall be set forth, at least in part in the following description. These will also become apparent to those skilled in the art upon examination of the following discussion or may be learned by the practice of the invention. The objects and the advantages of the invention may be realized and attained by means of the instrumentalities and in the combinations particularly pointed out in the appended claims.

In the description of this invention, the radiation emitters have been located on the first and second objects, and the sensors for these radiations have been located in fixed positions within the present time coordination system. It is considered that this arrangement of these emitters and sensors could readily be reversed. That is, the emitters could occupy the fixed positions in the present time coordination system and the sensors could be located on the first and second objects. The invention would operate in the same manner with either arrangement of sensors and emitters. For convenience, the description of this invention has placed the emitters on the moving objects and the sensors in fixed positions. This arrangement should not be considered to be a limitation on either the apparatus or method of this invention.

## Brief Description of the Drawings

The accompanying drawing figures illustrate a preferred embodiment of the present invention and, together with the description, serve to explain the principles of the invention.

Figure 1A is a block flow diagram of the optical mensuration and correlation system of the present invention showing the major components of this system, except for the means to automatically measure the position and orientation of the movable and second object.

Figure 1B is similar to Fig. 1A but includes the additional radiation emitters which will permit automatically measuring the position and orientation of the second object even during the movement of this second object.

Figure 2 is a perspective view illustrating the invention in use by a surgeon performing intracranial surgery on a patient, and showing a cursor on a display screen that marks the corresponding position of the tip of the probe (the first object) within the image of previously obtained model data corresponding to the cranium (the second object).

Figure 3 is a view of a sample display showing a position of the tip of the probe superimposed on previously obtained model data of an inside slice of a cranium and the showing reference points of the second object, as depicted in figure 1A, as triangles on the patient's skull.

Figure 4 is a schematic perspective view of a sample of one of the one-dimensional photodetectors which are useful in the practice of the present invention.

Figure 5 is a graph of the image intensity (manifested as a voltage or current) versus locations on the photodetector surface for a typical light detector which could be used by the radiation supported mensuration and correlation apparatus of the present invention.

Figures 6 and 7 are diagrams of the major steps performed by the computer to calculate the position of the probe (first object) with respect to the model of the inside of the cranium (the second object) and to display a cross-sectional image slice of the model of the inside of the cranium on a suitable display screen, such as a computer screen (CRT).

Figure 8 is a schematic view of radiation beams depicting an embodiment of this invention.

## Detailed Description of The Preferred Embodiments of This Invention

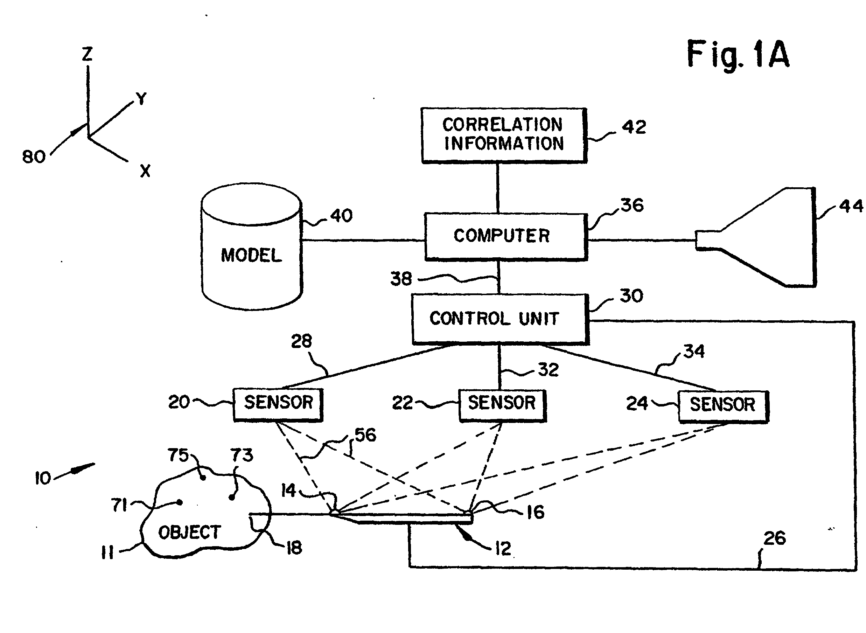

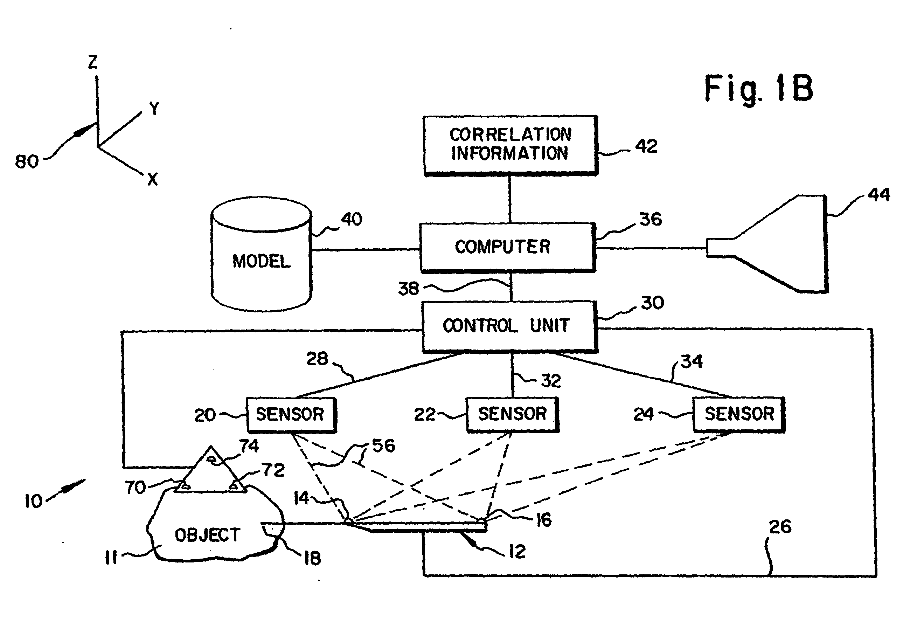

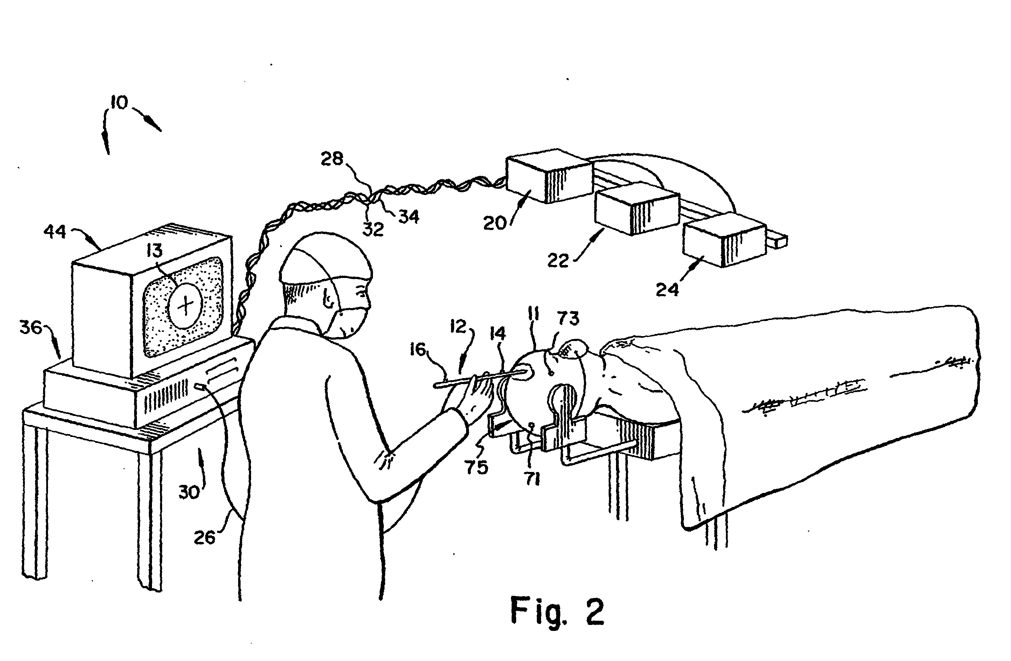

The radiation mensuration and correlation apparatus 10 of the present invention, as applied to a medical application, which is illustrative of one use of this invention, is shown schematically in Figure 1. It comprises a hand-held invasive probe 12 (first object) housing at least two radiation emitters 14 and 16 mounted collinear with one another and with the tip 18 of the probe 12. At least three remotely located, one-dimensional radiation sensors 20, 22, and 24 are mounted in fixed, spaced relationship to each other and are located at known positions with respect to a predetermined fixed coordinate system 80. The radiation sensors 20, 22, and 24 sense the radiation widely projected by the individual emitters 14 and 16 and generate electrical output signals from which are derived the location of the probe emitters 14 and 16 and, consequently the position and orientation of the probe tip 18 (which may not be visible to the surgeon because it is within the cranial cavity), with respect to the fixed coordinate system 80. In addition, where it is desired to determine the moving position of the cranium during the operation, the three sensors 20, 22, and 24 can be programmed to sense and derive the locations of other reference emitters 70, 72, and 74 on the second object 11 (Figure 1B) in the same manner as for the probe emitters 14 and 16. The role of these reference emitters on the second object is to automate the calculation of the relationships between the present time coordinate system of the model's image 13 (Figure 2) of the second object, the coordinate system of the sensors, and the local coordinate system of the second object itself 11.

A control unit 30 connected to the moveable probe 12 via a data line 26 and coupled to the remotely located sensors 20, 22, and 24 via data lines 28, 32, and 34, respectively, synchronizes the sensing of the five (exemplary) emitters and the differentiation between them. In that embodiment of this invention where the various emitters emit radiation in pulses, as by strobing them, the control unit is adapted to control the time multiplexing of the two emitters 14 and 16 on the probe and the three emitters 70, 72 and 74 on the cranium, controls the operation of the sensors 20, 22, and 24, and receives differentiatable data from these sensors as will be more completely described below. A coordinate computer 36, coupled to the control unit 30 by a data line 38, calculates the three-dimensional spatial location of the probe emitters 14 and 16 and consequently the position and orientation of the probe tip 18, and correlates those positions with data from correlation information 42 and from a model 13 of the second object 11 which has been previously stored electronically in an electronically accessible database 40. Finally, the computer 36 causes an associated cathode ray tube-monitor (CRT) to display the representation of the position and the orientation of the probe tip 18 with respect to the computer image 13 of the cranium 11 on display screen 44 (Figure 2) as will be more fully described below.

The probe 12 could be used without the cable 26, in that it could be coupled to the control unit 30 by employing distinctive modulation of the light emitters 14 and 16 instead of sequentially energizing (strobing) them, or by varying the wavelength or type of the radiation emitted therefrom. For example, the wave forms, color, or frequencies of each could be different. In the case of using sound radiation, the frequencies of the sound emitted by the different emitters could be varied so as to differentiate between them. The controller 30, by detecting the differences between different emitters, that is the wave form, color, frequency or other dissimilarity, of the emitted radiation, can determine to which emitter the sensors 20, 22, and 24 are reacting.

The fundamental mensuration and correlation apparatus 10 of the present invention has been illustrated in connection with aiding surgeons performing delicate intracranial surgery. This general use of this apparatus does not constitute this invention, nor is it a limitation thereon. This use will only serve to illustrate this invention. The remaining description continues to use such a surgical embodiment as illustrative, although many other surgical or other applications besides intracranial surgery are possible (for example, back or sinus surgery and breast biopsy). Moreover, the radiation mensuration and correlation apparatus 10 of this invention may be used for other purposes in many various medical or non-medical fields. In the described embodiment, the physical object 11 of interest, that is the second object in the generic application of this invention, is the head or cranium of a patient, and the model of the cranium is replicated using a series of parallel internal image slices (of known mutual spatial relationship) such as those obtained by means of computed tomography (CT) or nuclear magnetic resonance imaging (MRI). These image slices are then digitized, forming a three-dimensional computer model of the patient's cranium which is then stored in the electronically accessible database 40.

As shown in Figures 1A, 1B, 2, and 3 a surgeon places the tip 18 of the probe 12, that is the first object, at any point on or inside the cranium 11 of the patient. The position sensors 20, 22, and 24 detect the locations of the emitters 14 and 16 attached to the portion of the probe 12 that remains outside the patient's body. In order to accomplish this, the radiation produced by the emitters 14 and 16 must be "visible" to the sensors 20, 22, and 24. For that reason, more than two emitters may be placed on the first object so that the radiation from at least two of them will always be visible to the sensors. These emitters 14 and 16 are effectively point sources and radiate energy through a wide angle so that this radiation is visible at the sensors over a wide range of probe orientations and positions.

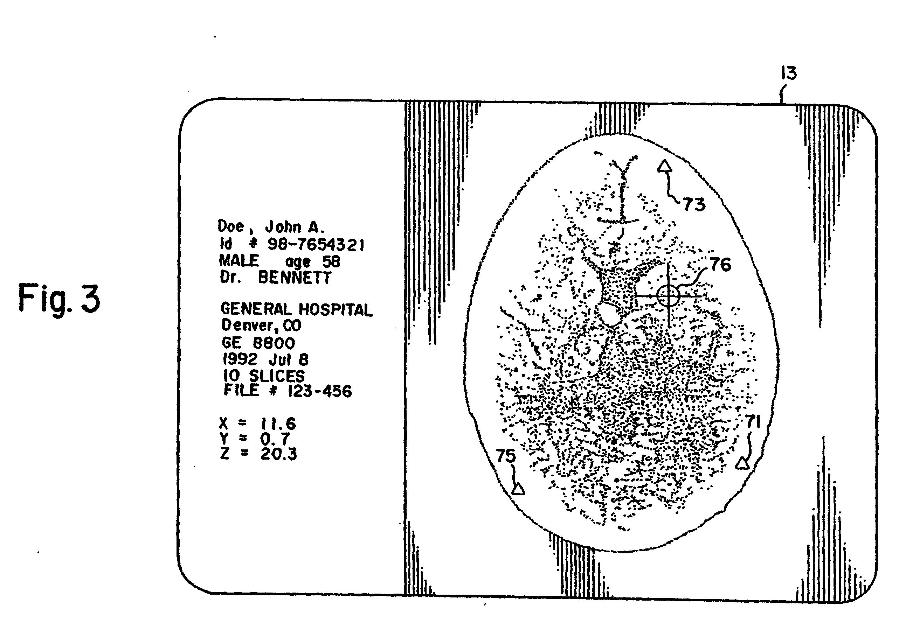

The sensors 20, 22, and 24, the control unit 30, and the computer 36 cooperate to determine the three-dimensional location of each emitter 14 and 16 within a coordinate system, and compute the coordinates of each emitter in the predetermined fixed coordinate system 80, in present time. The computer 36 can then calculate the position and orientation of the tip 18 of the probe 12 with respect to the predetermined fixed coordinate system 80, according to the locations of the emitters within the fixed coordinate system 80 and the dimensions of the probe, which dimensions had been placed into the memory (not shown) of the computer 36 beforehand. It should be noticed that the computer 36 can also easily compute position and orientation information about other specific locations on the probe (such as the vector from emitter 14 to the tip 18). Once the computer 36 has calculated the location of the probe tip 18 with respect to the fixed coordinate system 80, the computer 36 then uses the relationship between the model of the cranium, which had previously been obtained and stored in the database 40, and the fixed_coordinate system 80 to calculate the position and orientation of the probe tip 18 in relation to the model of the second object 11. Finally, the computer 36 displays a representation of the model-relative position and the orientation of the tip 18 on a display screen 44. In a simple form of the preferred embodiment of this invention, the computer 36 accomplishes this display by accessing a previously taken CT or MRI image slice 13 stored in the database 40 that is closest to the present time position of the probe tip 18, and then superimposes a suitable representation 76 of the tip 18 on the image 13 as shown in Figures 2 and 3. Thus, the surgeon knows the precise position and orientation of the tip 18 in the patient's cranium relative to the image data by merely observing the display screen 44. A most preferred form of the present invention can derive and display an arbitrary oblique cross-section through the multiple image slices of the MRI, etc, where the cross-section can be, for example, perpendicular to the probe orientation.

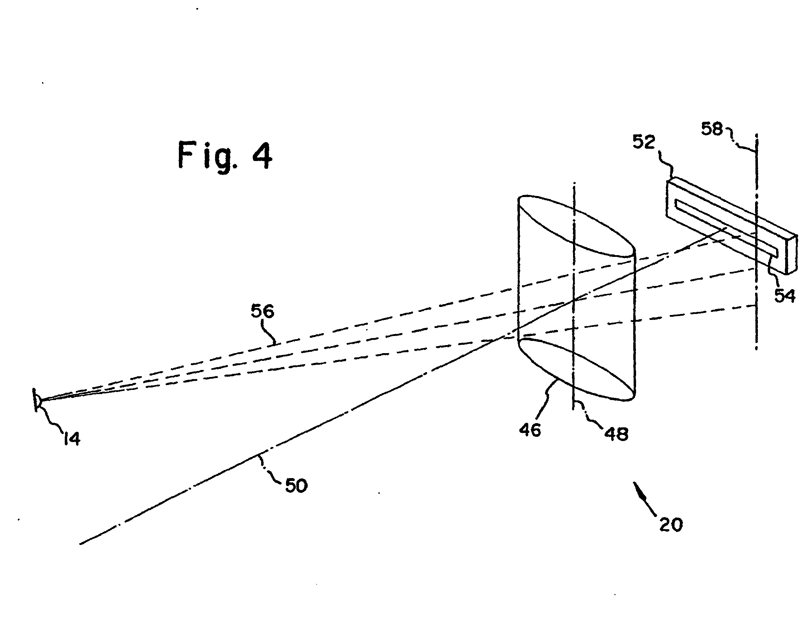

The details of the optical mensuration and correlation apparatus 10 of the present invention are best understood by reference to Figures 1 and 4 collectively. Essentially, the probe 12 supports the two radiation emitters 14 and 16, which are rigidly attached to the probe 12 at fixed, known distances from each other as well as from the probe tip. Since only two emitters are used here as being representative of the practice of this invention, the emitters 14 and 16 should preferably be collinear with the tip 18 of the probe 12 so that the computer 36 can determine uniquely the position and orientation of the tip 18 in three dimensions. Moreover, for reasonable measurement accuracy, the emitters 14 and 16 should preferably be at least as far from each other as the nearest one is from the tip 18. In any case, the geometrical relationship of the emitters 14 and 16 to each other and to the probe tip 18 must be specified to the computer 36 beforehand so that the computer 36 can compute the exact location of the tip 18 based on the locations of the individual radiation emitters 14 and 16. The use of three or more non-collinear emitters would not require that any two of them to be collinear with the probe tip. Three or more non-collinear emitters would permit the computer to compute full position and orientation information (yaw, pitch, and roll) for the probe. Although the invention is described as showing only a cursor locating the relative position of the probe tip 18, the invention can be modified to display a line or a shaped graphic or other icon to indicate the position and orientation of the probe 12. This would entail only the determination of additional points on the probe in the same way that the tip of the probe is located.

The two radiation emitters 14 and 16, as well as the additional radiation emitters 70, 72, and 74, can be, and preferably are, high intensity light emitting diodes (LEDs), which are preferably coordinated such that the emission for any one source is distinguishable from the emissions from the other sources. One such differentiation is to have the emitters time-multiplexed or strobed by the control unit 30 in a predetermined sequence such that only one light emitter is "on" or emitting light at any one time. The light emitted from any one of these emitters is detected by each of the three light sensors 20, 22, and 24, which then determines the location of each particular emitter in relation to the known positions of the sensors 20, 22, and 24 at the time it is strobed.

Each of the one-dimensional sensors 20, 22, and 24 used in the preferred embodiment 10 of the present invention can be identical to the others in every respect. Therefore, for the purpose of giving a detailed description of this embodiment, only the sensor 20 is shown and described in detail in figure 4 since the remaining sensors 22 and 24 are identical.

In Figure 4, the representative one-dimensional sensor 20 comprises a cylindrical lens 46 having a longitudinal axis 48 which is orthogonal to the optical axis 50 of the sensor 20. A linear radiation detector 52, such as a charge coupled device (CCD) with several thousand elements (or a similar device capable of linear positional radiation detection of a suitable "image"), is positioned in such a manner that the "optical" axis 50 passes through the center of the aperture 54 of the radiation detector 52 and such that the longitudinal axis of the aperture 54 is orthogonal to the longitudinal axis 48 of the lens 46. Radiation, such as light beams, 56 from the emitters 14 (and in the same manner emitters 16, 70, 72, and/or 74) are focused by the cylindrical lens 46 into a real image line 58 on the surface 60 of linear detector 52.

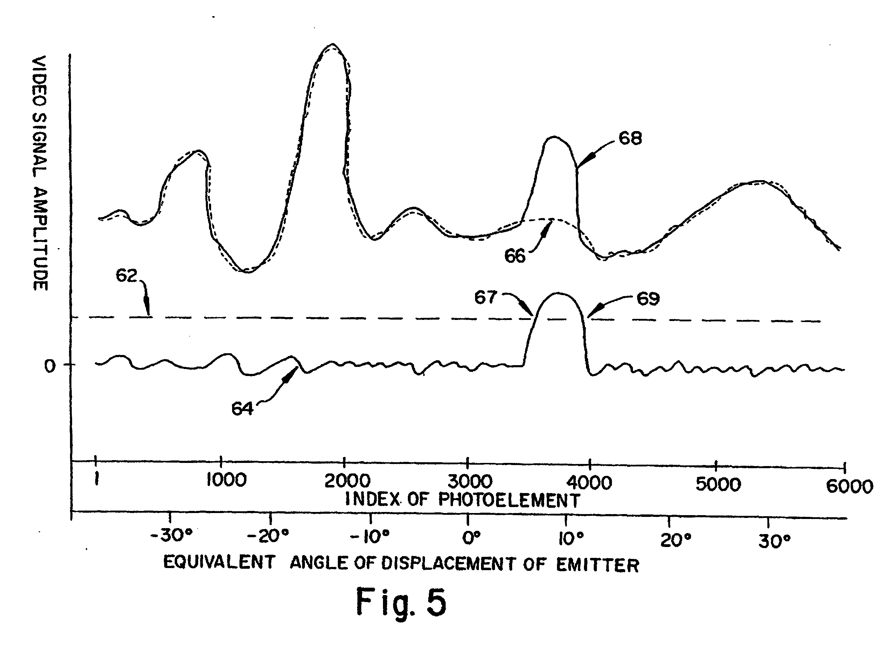

The detector, illustrated by a photodetector 52, then generates an output 68 (Figure 5) that is related to the position of a real image line 58 on the surface 60 of photodetector 52, thus characterizing the location of the image itself. That is, those elements or points of the photodetector 52 illuminated by the real image line 58 will generate a strong signal, while those not illuminated will generate none or very weak signals. Thus, a graph of image intensity (or signal strength) versus locations on the surface of the photodetector will resemble a signal peak curve 68 (see for example Figure 5). The "all-emitters-off" (or background) signal level 66 is never quite zero due to the effects of environmental radiation, such as light in the operating room, electronic noise, and imperfections in the photodetector. In any event, since the image of the illuminated emitter is focused into line 58, only the angular displacement of emitter 14 from the optical axis 50 in the plane of the longitudinal sensor axis 54 is measured by the sensor 52, hence the designation "one-dimensional sensor".

Thus, a single one-dimensional sensor 20 can only locate the plane on which a radiating emitter 14 lies. The detector 20 cannot, by itself, determine the unique point in space on that plane at which radiating emitter 14 is located. To precisely determine the location in space of the radiating emitter 14 requires at least three such sensors positioned in spaced relationship to each other, since the intersection of three planes defined by the three sensors, respectively, are required to define a single point in space.

To locate the position of one particular radiating emitter, such as 14, the sensors 20, 22, and 24 are mounted so that the optical axes of their lenses 48 are not all parallel and no two of such axes are collinear. In a preferred embodiment of this invention, two light sensors, such as sensors 20 and 24 in Figure 2, are situated so that their respective axes 48 (Figure 4) are in parallel, spaced relationship, and the third detector 22 is situated between and equidistant from the other two detectors, but with its axis 48 perpendicular to the axes of the other two. That is, the sensors 20, 22, and 24 should be arranged along a line or arc (Figure 2), such that each sensor 20, 22, and 24 is generally equidistant from the center of the volume in which the measurements are made, equally spaced from each other, and all aimed at the center of the measurement volume. Suppose for example that the sensors 20, 22, and 24 are arranged along a horizontal arc and the optical axes of all sensors are oriented horizontally. Then the middle sensor should be oriented so as to measure the angular elevation of the radiation emitters as described above. The two outer sensors measure the horizontal angle (azimuth) relative to the fixed coordinate system 50. Data from the outer sensors are used to stereographically calculate both the horizontal position and distance from the sensors as will be more fully described below.

The accuracy of three-dimensional measurement depends on the angle formed between the optical axes of the outer two sensors 20 and 24, where the emitter to be measured is at the vertex of the angle. Accuracy will improve as that angle approaches a right angle. At least three of the several possible sensors 20, 22, and 24 must be spaced so that the desired measurement volume is completely within their field of view which can be accomplished by making the focal length of the lens 46 short enough to provide coverage of the entire desired field of view. In another embodiment of this invention, additional sensors, which may be substantially identical to sensors 20, 22, and 24, could be used to provide more viewpoints, to broaden coverage of the field of view, or to enhance measurement accuracy.

While this process of detecting a given radiating emitter, such as 14, can determine the exact location of the radiating emitter, it cannot by itself determine the particular orientation and position of the probe or its tip 18 in three-dimensional space. To do so with only two emitters requires that both emitters 14 and 16 be collinear with the probe tip 18, as described above. Also, the distances between each emitter, 14 and 16, and the probe tip (as well as the distances between the emitters 14 and 16 themselves) must be known and loaded into the memory of the computer 36 before the computer 36 can determine the position and orientation of the probe tip 18 from the locations of the emitters 14 and 16 in the fixed coordinate system 80. Consequently, when each of the radiation emitters 14 and 16 is rapidly turned on in sequence, or strobed, the sensors 20, 22, and 24 can detect the exact location of each emitter in turn. Thus computer 36 can determine the exact position and orientation of the probe, and therefore its tip 18. Since only one of the radiation emitters 14 or 16 is on at any one time, the detectors 20, 22, and 24 locate the location of that particular illuminated emitter individually. If the strobe rate, that is, the frequency at which the emitters 14 and 16 are turned on and off in sequence, is fast enough, the detectors 20, 22, and 24 can, for all practical purposes, determine the position and orientation of the probe 12 and its tip 18 at any instant in time, and therefore can follow the movement of the probe tip in present time, that is during the time that the probe tip is actually moving. In other words, this system can simulate the movement of the probe tip on the previously taken image in present time during the surgical procedure.

The sensors 20, 22, and 24 need only distinguish which of the radiation emitters 14, 16, 70, 72, or 74 is on at any one time. In the preferred embodiment 10 of the present invention, this function is accomplished by strobing each of the emitters in sequence, as described above. However, other methods can be used to allow the sensors 20, 22, and 24 to distinguish the respective radiation emitters 14, 16, 70, 72, and 74 from one another. For example, different wave lengths (colors) of light, or different frequencies of sound, could be used in conjunction with detectors capable of distinguishing those particular different radiations.

Alternatively, it is one aspect of this invention to modulate each of the respective radiation emitters 14, 16, 70, 72, and 74 with a unique wave form or pulse train. This means of differentiating between the different emitters is believed to be novel and unique to the instant invention. If such different wave forms or pulse trains are used to differentiate the different emitters, one may also transmit additional information on these wave forms, such as for example the temperature of the particular structure being contacted by the probe tip 18. It is within the scope of this invention to provide means readily accessible to the surgeon or other operator to engage or disengage the taking of such additional information and the transmission thereof by the unique wave forms or pulse trains radiated by the respective emitters. Under these circumstances, the control unit 30 or computer 36 will be designed to demodulate the wave form to determine to which particular emitter the sensed signal belongs, and to decode the additional information being transmitted.

Numerous other methods for distinguishing the radiation emitters are possible. Therefore, the present invention should not be regarded as limited to the particular strobing method shown and described herein, but is generic to the use of any means to differentiate between the different emitters.

Conventional or unique auto-focusing or multiple-lens radiation detection may be integrated into the sensors 20, 22, and 24 to improve the performance of the system. However, the simple, fixed-focus optics shown and described herein and shown in Figure 4 for one sensor provide a good level of performance if the working range of the probe is restricted. Even if the real image of an emitter, such as 14, is somewhat out of focus on the detector 52, the angular measurement of the image is still usable. A usable measurement for each of the sensors 20, 22, or 24 to generate will be any of the following: (1) the position of the detector element with peak intensity, (2) the intensity-weighted average (centroid) of all over-threshold elements, or simply (3) the average of the minimum and maximum elements where the intensity is over some threshold. The detector 52 should be placed at the focal distance for the farthest typical operating distance of the radiation emitters. Closer emitters will form slightly defocused images 58, but they require less precise angular measurement for a given distance accuracy. Furthermore, their de-focused real images are brighter, which increases the brightness gradient at the edges of the image.

As described so far, the real image 58 of the currently activated emitter must be significantly different from (for example brighter than) the rest of the radiation falling on the sensor 52. Otherwise, other lights or reflective surfaces in the field of view of the sensors will hinder the detection of the emitter's real image. Therefore, it is desirable to include in the apparatus, circuitry to subtract the background radiation received by the sensors from other, ambient, sources.

This _per se_ known circuitry enhances use of the invention where the sensors are required to detect the radiation emitters against relatively bright backgrounds. While the radiation emitters are all momentarily extinguished, the one-dimensional data from each sensor are saved in a memory. This can be done in an analog delay line or by digitally sampling the output signal and storing it in a digital memory. Then, as each emitter is "viewed" sequentially, the saved data are subtracted from the current data generated by the currently radiating emitter. If the background data are stored digitally, the current data are also digitized, and the stored background data are digitally subtracted from the current data.

A graphical representation of the radiation intensity of the image or, equivalently, the generated output voltage amplitude for each element in a row of detecting elements, is shown in Figure 5. The graph depicts typical background image intensities 66 with all emitters off, the intensities 68 with one radiation emitter on, and the element-by-element difference 64 between the intensities with the emitter off and those with it on. The measurements will likely contain some random noise, electronic or otherwise, and two consecutive measurements for a given sensor element may differ slightly even where the background is unchanged. Therefore, the differential intensities 64 between two consecutive measurements also contain some random electronic noise. However, the two measurements differ substantially only at the location of the radiation emitter image, and this difference exceeds the threshold level 62.

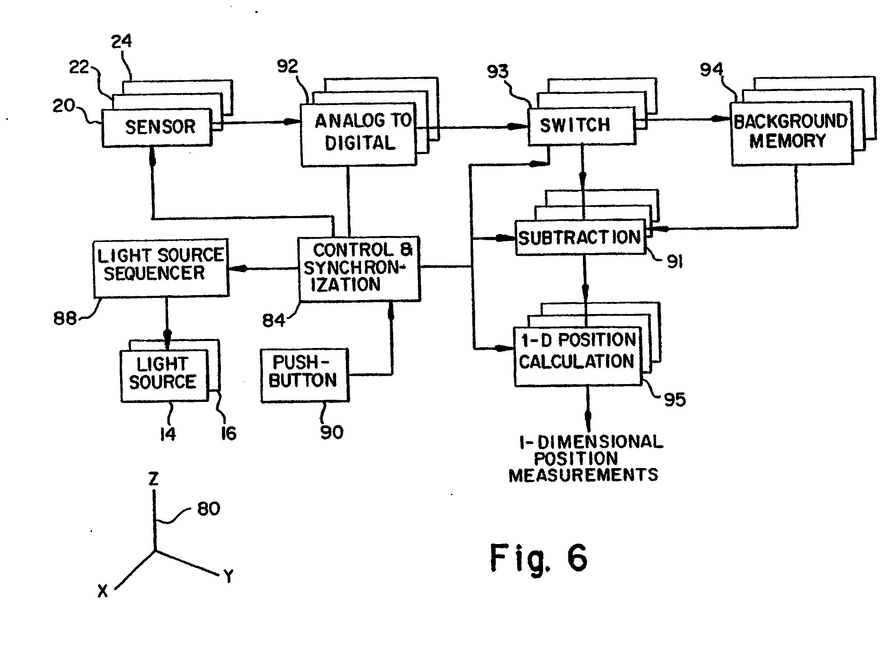

The details of the structure and operation of the control unit 30 are best seen in Figure 6. Specifically, control unit 30 (see Figure 1A and 1B) supplies power to the radiation emitters 14, 16, 70, 72, and 74 and the radiation sensors 20, 22, and 24. A control and synchronization unit 84 and radiation source sequencer 88 (where a strobed radiation sequencing is used) time-multiplexes or strobes the radiation emitters individually, as described above, so that the position and orientation of the probe tip 18 (Figure 1) can be determined from the signals received from the sensors 20, 22, and 24. The angular data signals received from the sensors 20, 22, and 24 are converted by an analog-to-digital converter 92. Actually, three analog-to-digital converters are used, as shown in Figure 6, but only one is labeled and described herein for brevity, since the other two analog-to-digital converters are substantially identical and are used to convert the signals from the other sensors 22 and 24.

The control and synchronization unit 84 also controls three switches, of which switch 93 is typical, which store all digital data received from the sensors 20, 22, and 24 when the radiation emitters 14 and 16 are off and stores these data into a background memory 94. Then, when the radiation emitters 14, 16, 70, 72, and 74 are illuminated in sequence by radiation source sequencer 18, the synchronization and control unit 84 changes the state of switch 93 which then redirects the data from the three sensors 20, 22, and 24 to a subtraction unit 91. The subtraction unit 91 subtracts the background data from the emitter radiation data, thus resulting in a signal which has been relatively freed from the background signal 66 (Figure 5) since the fixed pattern noise has been subtracted from the signal.

As shown in Figure 6, which should be considered in conjunction with Figure 5, a 1-D (one-dimensional) position calculation unit 95 determines the location of the real image line 58 on the CCD sensor 52 (Figure 4) by measuring the locations of the edges 67 and 69 of the signal blip 68 (Figure 5) generated by the CCD sensor based on a predetermined threshold signal level 62. The 1-D position calculation unit 95 then averages the distance between the two edges to find the center of the signal peak 68 as shown in Figure 5. This method of determining the center of the signal peak is per se well known in the art and need not be described in further detail. Moreover, numerous other methods of determining the location of the signal peak or its centroid are known in the art and will be obvious to those of ordinary skill in the art. The method used depends on the signal characteristics of the radiation sensor used as well as the characteristics of the lens system used to focus the radiation onto the surface of the detector, in addition to other parameters. Those practicing this invention with the various alternatives described herein would have no trouble selecting a signal detection algorithm best suited to the particular characteristics of the sensors and the particular radiation being used.

Finally, the control unit 30 (Figure 1) transmits the radiation data to the computer 36. That is, when the computer 36 is ready to compute the current location of the currently radiating emitter, such as 14, the latest angular data from all sensors 20, 22, and 24 are provided for analysis. If the sensors generate data faster than the control unit 30 can process them, the surplus angular data are simply discarded.

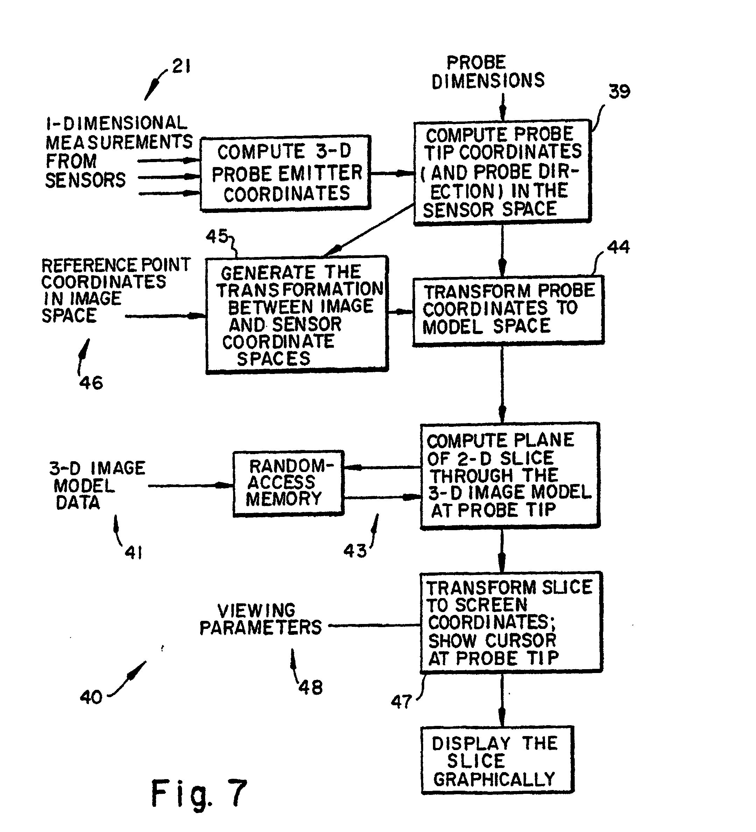

The operation of the computer 36 is most advantageously set forth in Figure 7. The computer 36 calculates one-dimensional positions for each radiation emitter such as 14 or 16, based on the location of the signal peak from each respective sensor 20, 22, and 24. These one-dimensional angular position measurements are then used to determine the three-dimensional spatial coordinates of the emitters 14 and 16 and thus for the position and orientation of the probe 12 relative to the predetermined fixed coordinate system 80 by coordinate transformation methods which are per se well-known in the art. The output signals from the computer 36 can be in any form desired by the operator or required by the application system, such as XYZ coordinate triples based upon the predetermined fixed coordinate system 80.

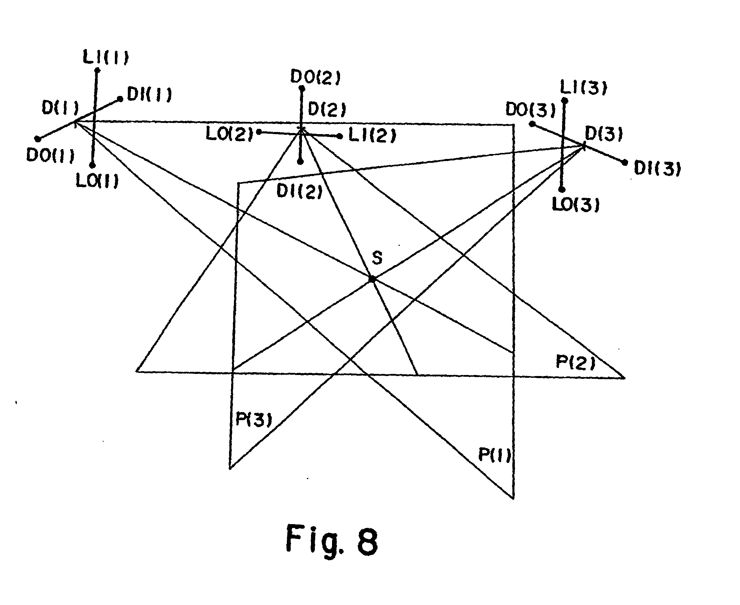

Figure 8 and the following paragraphs describe in detail how the location of a single radiation emitter, such as 14, is computed from the data derived from the sensors 20, 22, and 24. The following description applies to these three sensors 20, 22, and 24 only. If there are more than three such sensors, the calculation can be performed using any three or more of the sensors. Furthermore, if more than three sensors are used, the average of the points calculated from all combinations of three sensors could be used to increase accuracy. Another option is to use the point calculated from the three sensors closest to the radiation emitter 14 or 16. The following parameters are considered to be known XYZ constants:

D0[i], one endpoint of each linear photodetector i;
D1[i], the other endpoint of linear photodetector i;
L0[i], one endpoint of the axis of each lens i; and
L1[i], the other endpoint of the axis of lens i.

Each sensor generates T[i], a parametric value between 0 and 1 indicating where the peak or center of the line image of the emitter intersects the line segment between D0[i] and D1[i]. The XYZ coordinates of point S are to be calculated, where S is the location of the radiation emitter. For a CCD radiation detector array, T[i] is the index of the element on which the center or peak of the image falls divided by the number of elements on the detector array.

The three-dimensional coordinates of the above points are all referenced to a predetermined fixed coordinate system 80. The cylindrical lens and linear photodetector do not directly measure the angle A of the radiation emitter about its lens axis; rather, they measure a value T[i] linearly related to the tangent of that angle:

$$\tan(A) = C * (2 * T[i] - 1),$$

where C is a constant of proportionality that is related to, and determined empirically by, the dimensions of a particular system.

The three-dimensional location of the image line on the linear photodetector is:

$$D[i] = (1 - T[i]) * D0[i] + (T[i]) * D1[i]$$

If the lens is ideal, then S also lies in plane P[i]. In reality, the point D[i] might have to be computed by a non-linear function F(t) that corrects for non-linear aberrations of the lens or the photodetector:

$$D[i] = (1 - F(T[i])) * D0[i] + (F(T[i])) * D1[i]$$

Function F(t) could be a polynomial in variable T, or it could be a value interpolated from an empirically determined table.

P[i] is the unique plane determined by the three points D[i], L0[i], and L1[i], which are never collinear. S is the point of intersection of the planes P[1], P[2], and P[3] determined respectively by sensors 1, 2, and 3. S is a unique point if at least two sensor lenses longitudinal axes 48 are not parallel and if no two lens axes 48 are collinear. The intersection point is found by finding the common solution S of the three equations defining the planes P[i]. Once the location S of each of the probe's radiation emitters is computed, the location of the probe's tip 18 can be calculated. The method of making such a determination is well known using the teaching of analytic geometry and matrix manipulations.

If M is a linear transformation describing the relationship between a point R in the image coordinate system and a point S in the fixed coordinate system, then:

$$R * M = S.$$

If $M^{-1}$ is the inverse of M and if S is a point in the fixed coordinate system, then the point R in the image coordinate system corresponding to S is:

$$S * M^{-1} = R.$$

Now, suppose that the second object is moved in the mensuration coordinate system. This can be described by a linear transformation U where the coordinates S of a point are mapped to the coordinates S':

$$S * U = S'$$

Then the old value of M above must be multiplied by U in order to correct the relationship between the point R in the image coordinate and the corresponding point in the mensuration coordinate system because of the relative movement of first object with respect to the second object:

$$R = S' * U^{-1} * M^{-1}$$

The preliminary steps required before practicing the method of the invention are now described. Then, after fully describing these preliminary steps, the detailed steps of the method of the optical mensuration and correlation apparatus are described.

Use of the invention takes place in three phases: the imaging phase, the correlation phase, and the normal operation phase. The imaging phase precedes the normal operation of the present invention. During the imaging phase, a scan of the body of the second object of interest is used to build a three-dimensional geometrical model. In the preceding description, the second object was the head of a human intracranial surgical patient because the invention is advantageously used in stereotactic neurosurgery. Accordingly, the three-dimensional model comprises digital data from a series of internal cross-sectional images obtained from computed tomography (CT), magnetic resonance (MRI), ultrasound, or some other diagnostic medical scanner. In any case, the image data are stored in a suitable, electronic memory 40 which can be accessed later by the computer 36. The data are considered to be stored as a series of parallel two-dimensional rectangular arrays of picture elements (pixels), each pixel being an integer representing relative density. If the object is relatively rigid, like a human head, this three-dimensional model may be created at some time before the correlation and operational phases of the invention and possibly at another location.

Also, during the imaging phase, at least three non-collinear reference points 71, 73, and 75 (Figures 2 and 3) must be identified relative to the object 11. These may be represented by ink spots, tattoos, radiopaque beads, well-defined rigid anatomical landmarks, locations on a stereotactic frame, sterile pins temporarily inserted into rigid tissue or bone of a surgical patient, or some other reference means. The coordinates of these reference points are measured and recorded relative to the coordinate system of the imaging device. One way to accomplish this is to capture the reference points as part of the previously made three dimensional model itself. For example, radiopaque pins could be placed within the image planes of diagnostic CT slices; the pin locations, if not automatically detectable from their high density, can be identified interactively by the surgeon using a cursor on the computer display of the CT slices. See Figure 3.

The initializing of the position and the orientation of the second object, the patient's cranium, is well known in this art. The instant invention departs from this well known operation to add radiation emitters which have a known and exact spacial relation to these fiducial markings. These additional radiation emitters must then be programmed or otherwise activated for use in a particular manner in order to practice the instant invention. They must be programmed to radiate at some frequency during the surgical procedure which is in progress during present time so that the position and orientation of the second object will be available to the surgeon at all relevant times and so that this position and orientation can be repeatedly and automatically updated in order for the system to revise the specific selected scan slice to superimpose the position and the orientation of the first object on in a correct depiction of the actual relative positions and orientations of both the first and second object in present time during the surgical procedure.

The initial correlation mode immediately precedes the normal operational phase of the present invention and must take place in the operating room. During this initial correlation phase, the instant system accesses the data of the three-dimensional geometrical model of the patient (or other object), including the reference point (fiducial marker) coordinates which were recorded earlier, that is previously. Next, the surgeon may place the tip of the probe 18 at each of the reference points 71, 73, and 75 on the patient, in turn. This sequence of operations may be directed by the computer program. In the alternative, the system of this invention provides these data automatically by the radiation from the emitters 70, 72, and 74 being received by the sensors directly and automatically without special intervention by the surgeon. Either of these procedures establish an initial relationship between the locations of these reference points in the model coordinate system and their current physical locations in the fixed coordinate system 80. However, the preferred determination of this initial position and orientation also carries on during the whole of the surgical procedure and therefore is capable of substantially continuously updating the position and orientation of the second object and relating it to the current position and orientation of the probe. In turn, this establishes a linear mathematical relationship between all points in the model and points in the coordinate system 80. Thereafter, when the patient is moved relative to the sensors, the prior art must establish a new relationship by again digitizing the reference points 71, 73, and 75 within the coordinate system 80. That is, the correlation phase must be repeated. Again, the system of this invention uses the emitters 70, 72 and 74 to accomplish this automatically. For this reason, the automatic tracking of the position of the head, or the second object whatever that is, which is described below and which overcomes this problem, is an essential, significant feature of the present invention.

Since the position and orientation of the head is initially and substantially continually thereafter correlated with the model, the surgeon can relate any locations of interest on the diagnostic images with the corresponding physical locations on this patient during the operation, and vice versa. These include locations accessible to the probe tip 18 but not necessarily directly visible to the surgeon.

Having described the function and purpose of the preliminary steps, the detailed method of the present invention is more easily understood. As shown in Figure 7, the position data 21 of the probe emitters generated by the sensors and control unit are converted into three-dimensional coordinates relative to the predetermined fixed coordinate system 80 of the sensors. Using dimensional parameters describing the relationship among the probe emitters and the probe tip, the computer determines the coordinates of the probe tip in a step 39. During the initial correlation phase, the probe tip may be placed at each of the reference points 71, 73, and 75 in turn. Alternatively, in accord with a preferred aspect of this invention, the emitters 71, 73 and 75 are located by the sensors and the correct position and orientation of the second object is thereby determined. The coordinates of the second object in the fixed coordinate system along with their coordinates 46 in the image coordinate system determine a unique linear transformation relating the two coordinate systems in a step 45. This is a per se known calculation in analytic geometry and matrix mathematics.

As noted above, a more automated and direct method of determining the location of the second object is to directly read the locations of the fiducial points 71, 73 and 75 by the fixed sensors 20, 22 and 24. This can be accomplished by placing radiation emitters 70, 72, and 74 (Figure 1B) at those reference points (or in a known fixed spacial relationship to those reference points 71, 73, and 75). The emissions of these emitters can then be read directly by the sensors 20, 22, and 24, and thus the computer can then automatically determine their locations relative to the predetermined fixed coordinate system 80 of the sensors. Thus the position and orientation of the second object, the cranium in the preferred embodiment of this invention, can be automatically and substantially continuously determined. With the position and orientation of the second object being at least frequently, if not substantially continuously, updated, the position of the first object, the probe, which is also determined at least frequently, if not substantially continuously, can then be updated in relation to the second object at the same frequency. Since the position and orientation of both the first and the second objects are each at least frequently determined and updated in relation to the fixed coordinate system 80, the position and orientation of each of these first and second objects can then be determined relative to each other, by indirect, but well known, calculations which are easily carried out in short order by a computer.

It has been stated herein that the position and orientation of the second object, the cranium, can, according to this invention, be determined continuously or at least frequently. The frequency at which the position and orientation of the second object is determined is a function of the desires of the operator of this system and the frequency at which the radiation emitters and the sensors can be operated. In the case where the emitters all emit the same wave length of radiation and the sensors all sense this same wave length of radiation, the differentiation of the emissions of the several emitters is followed in a sequential pattern. Thus, in this embodiment of this invention, the emitters will emit radiation in sequence, for example 14, then 16, then 70, then 72 and then 74. The sensors will have been programmed to identify a signal with an emitter as a function of when the signal is received.

In this embodiment of this invention, the position and orientation of the first object, the probe, is determined with the same frequency as is the location and the orientation of the second object, the cranium, because all of the emitters radiate in sequence. However, the system can be programmed so that the emitters 14 and 16 fire more or less frequently than the emitters 70, 72 and 74. Under these conditions, the position and orientation of the first object and of the second object will be determined at different individual frequencies, that is at the same frequency as the frequency of the radiation from their respective emitters. It will therefore be clear that the frequency of determination of the location of any given emitter, and therefore the determination of the position and orientation of these first and second objects, is, because of the instant invented system, for the first time entirely controllable by the programmer or the operator, within the capabilities of the system operating the emitters.

However, it should be understood that the position and orientation of the first and/or second objects can be determined in a substantially continuous manner. In this embodiment of this invention, each emitter will radiate a different wave length or wave form or frequency pulse of radiation. Therefore, the radiation emitted from each emitter is simultaneously distinct from the radiation emitted from the other emitters. Under these conditions, the location of each emitter can be determined continuously by a set of sensors which is tuned to the specific, different radiation of each emitter. Therefore, the location of each emitter can be determined continuously, whereby the position and orientation of either or both of the objects can be calculated by the computer from these continuous locations of the different emitters.

While it is a significant distinction of this invention from the prior art that:

in the prior art:

the first object is intended to be moved and the second object is intended to be stationary; and

position and orientation of the first object is frequently determined, but the position and orientation of the second object is only determined at the start of the operation and at any time that the second object, the cranium, which is intended not to be moved at all during the operation, is known by the surgeon to be moved;

whereas

according to this invention:

the first object is intended to be moved, and the second object is not intended to be rigidly immobilized in place, or, put another way, the second object is permitted to move and is even expected to move; and

the position and the orientation of the first object is frequently determined, and the position and orientation of the second object is also frequently determined. The position and orientation of these two objects may be determined at the same frequency or at different frequencies (or even continuously) as desired by the operator.

In preferred embodiments of this invention, the position and orientation of the second object will be determined from one hundredth to ten times, most preferably from a quarter as often to four times, as often as the frequency at which the position and orientation of the first object is determined. As a general proposition, there is no limit on the relationship between these frequencies of measurement. The preferred relationships set forth herein are illustrative and not limiting. The frequency of each measurement is dependent on the amount of movement which is allowed and is intended to be shown on the CRT. The upper limit on this frequency is determined by the ability of the emitters to be distinguished. There is no lower limit.

In the instant specification, the emitters have been described as being on the first and second objects and being movable therewith, and the sensors have been described as being in a fixed relation to the coordinate system. While this is the preferred system, it is by no means the only configuration of the system of this invention. It is also within the scope of this invention to provide the emitters in fixed relationship to the coordinate system, and the sensors on the first and second objects, respectively. The wiring may be somewhat more cumbersome in this configuration, but that should not detract from the viability of such a reversal.

This invention has been described with reference to a first and a second moving object, and the determination of each of their absolute and relative positions and orientations in a fixed coordinate system. It will be clear that this same system applies to more than two objects. In fact, the position and orientation of any number of objects can be determined, both absolutely with respect to the coordination system, and relatively with respect to each other, by the practice of this invention. Thus, when this specification and the claims appended hereto speak of a first and a second object, these can be two out of any number of total objects. This number is merely illustrative of the practice of this invention and is in no way limiting thereon.

Thus, the preferred system of this invention performs the three primary tasks of this invention, preferably, but not necessarily, simultaneously:

the absolute position and orientation of the second object, the cranium, in the fixed coordination system is determined at least very frequently;

the relationship between the absolute position and orientation of the second object with respect to the previously taken images of that object, particularly the inside structures of that object, is determined at least very frequently; and

the absolute position and orientation of the first object, the probe, is determined at least very frequently. The accomplishment of these three tasks then permits the computer to accomplish the three essential secondary tasks of this invention:

to calculate the position and orientation of the first object, the probe, in relation to the second object, the cranium, even though the first object, or a portion of it, is out of the line of sight of either the surgeon or the sensors;

to select the appropriate slice of the previously taken model of the interior of the second object which corresponds to the present time position and orientation of the first object in relation to the present time position and orientation of the second object; and

to display the appropriate slice of the previously taken image of the second object with the present time position and orientation of the first object correctly depicted thereon.

Both the initial and the continual correlation determinations can be automatically initiated and updated by the computer 36 in some predetermined timed sequence or continuously. In fact, according to the most preferred aspect of this embodiment of this invention, the correlation phase is frequently, briefly from time to time, or even continuously, repeated, interspersed in between measurements in the operational phase or conducted simultaneously with the operational phase of the practice of this invention for the purpose of recalculating the linear transformations M and M' when the second object (such as a surgical patient) moves relative to the sensors.

During normal operation, the tip coordinates are transformed in a step 44 using the transformation computed in step 45. The new transformed coordinates, relative to the image coordinate system, are used to determine the plane of some two-dimensional cross-section through the three-dimensional image model 41 accessible in the accessible memory 43. The simplest method is simply to choose the existing diagnostic image plane located closest to the probe tip's coordinates relative to the model coordinate system.

In any case, a step 47 transforms the two-dimensional cross-sectional slice to a screen image and places a cursor on it to mark the location of the probe tip superimposed in the image. Scaling and viewing parameters determine how the image is displayed. Because the surgeon may not be able to simultaneously view the patient (object) and the computer display screen, the step 47 should be controlled by the surgeon, such as for example by placing an activating button on the probe. Pressing the button can be the signal for freezing the image and the depicted position and orientation of the probe tip marker at that instant on the display screen.

In a more complex embodiment of this invention, the computer system could generate and display on the screen a cut-away view at an arbitrary angle, for example, perpendicular to the direction the probe is pointing, using the data from multiple image slices. In simpler cases, the computer simply displays any one or more convenient image slices through the location of the probe tip. For example, the displayed slice might simply be the original CT slice which includes the location of the probe tip, or is closest to that location. In any case, the computer then causes the image a cursor at the current position of the probe tip to be displayed on this previously taken image of a slice through the second object.

An alternative means, to record the location of the reference points in the coordinate space of the imaging apparatus during the imaging phase, employs an additional, separate instance of the three-dimensional position mensuration probe, sensors, control unit, and computer of the present invention. In order to implement this embodiment of this invention, the additional sensors are permanently attached directly on the imaging apparatus. The additional probe measures the location of the reference points at the time of imaging, and the additional control unit and computer determines and records their locations relative to the coordinate system of the imaging apparatus. The advantage of this approach is that the fiducial markers, that is the landmarks or reference pins, need not be within the limited cross-sectional slices visible to the imaging device.

As an alternative to true three-dimensional images, standard x-ray radiographs from several distinct directions can be used to construct a crude model in lieu of the imaging phase described above. Radiographs from two or more directions are digitally scanned, and four non-coplanar reference points on them are identified with a cursor or light pen. In a correlation phase similar to that described above, these four points on the patient are digitized just prior to surgery. Then, during surgery, the location of the probe tip is projected onto the digitized computer images of the two-dimensional radiographs where the projection is uniquely defined by mapping and transferring the reference point coordinates from the model coordinate system to the fixed sensor coordinate system.

In a further embodiment of this invention, a videotape recording of the computer screen (as well as the direct view of the surgeon and patient) is used to help document the performance of the instant procedure. Radiation emitters may be present on more than one standard surgical tool such as the microscope, scalpel, forceps, and cauterizer, each of which thereby becomes, in effect, a probe. These emitters should be differentiated from each other in the same manner as aforesaid.

The method and apparatus of the optical mensuration and correlation apparatus 10 of the present invention has been completely described. While some of the numerous modifications and equivalents of the system of this invention have been described herein, still other modifications and changes will readily occur to those of ordinary skill in the art. For instance, the preferred embodiment described herein uses visible light, since human operators can readily observe if the light sources are operative or whether they are causing troublesome reflections. Clearly, other wavelengths of electromagnetic radiation could be used as well . Non-visible light, such as infrared or ultra-violet light, would have the advantage of not distracting the surgeon with flashing lights. Ultra-sound could be used conveniently. Other modifications to the detector "optics" and lenses are possible which would change, and possibly improve, the image characteristics on the detectors. For example, toroidal lenses could be used which are longitudinally curved along an arc with a radius equal to the focal length of the lens. Similarly, the surfaces of the photodetectors could also be curved, thus allowing the images of distant light sources to remain in sharp focus, regardless of their positions. Numerous enhancements of the digital data are possible by suitably programming the computer.

The most preferred aspects of this invention use electromagnetic radiation, and especially visible light, as the radiation from the emitters. This use of light for this function is a major improvement over the use in the prior art of audible sound emitters and detectors. However, prior art systems which are based on the use of sound emitters can be reprogrammed to carry out the operations to substantially continuously recorrelate the position and orientation of the second object during the surgical procedure, as they have been described herein. Thus, the movement of the second object can be at least frequently, if not continuously, tracked using sound emitters and detectors and suitable temperature compensation techniques. In this last regard, the aforementioned ability of the instant system to determine and transmit the temperature of the probe tip can be used to good advantage when using sound as the radiation of choice. The fact that the use of electromagnetic radiation, particularly light, emitters and sensors is an improvement over the use of audible sound emitters and sensors is not intended to be a limitation of the practice of continuously or frequently following the movement of the first or the second objects. That is an invention in and of itself using any emitter-sensor pair.

It should be understood, however, that the transmission between emitters and sensors operate differently, and measure different things when electromagnetic radiation is used as compared to the use of sound, audible or ultrasonic. In the case of electromagnetic radiation, what is being measured is the angle that the radiation path makes between the emitter and the sensor relative to some arbitrary fixed line. By measuring all of these angles of the rays between the emitters and the sensors, conventional analytic geometry solutions will locate the points in space where the various emitters are. On the other hand, when sound radiation is used, what is being measured is the distance between each of the emitters and the sensors. Again, conventional analytic geometry solutions will precisely locate the point in space which is occupied by each emitters/sensor. While the casual observer, or the operator of the systems of this invention will not observe any difference in result, there is a marked difference in the way that result is achieved, and therefore this will necessitate a difference in the manner in which this system is programmed.

## Claims

1. A medical system (10) for determining the present time location and orientation of a moveable first object (12) with respect to a second object (11) and for graphically indicating the corresponding position and orientation of said first object (12) on a previously taken image of said second object (11), which comprises:

> a present time three-dimensional fixed coordinate system (80);
> at least three electromagnetic or acoustic radiation sensor means (20, 22, 24) in fixed spatial relationship to said present time three dimensional fixed coordinate system (80) which are spaced from said objects (11, 12);
> said first and second objects (11, 12) located within said fixed coordinate system (80);
> at least three non-collinear fiducial markers (71, 73, 75) in fixed spatial relationship to said second object (11) and at known coordinates in said fixed coordinate system (80);
> a three dimensional local coordinate system which is fixed in relation to said second object (11);
> previously taken three-dimensional image data which geometrically describe said second object (11) including said fiducial markers (71, 73, 75);
> at least two spaced apart electromagnetic or acoustic radiation emitter means (14, 16) disposed on said first object (12);
> first movement means to move said first object (12) relative to said second object (11) and to said fixed coordinate system (80);
> means to distinguish electromagnetic or acoustic radiation emitted from any one radiation emitter means (14, 16) from radiation emitted from all of the other radiation emitter means (14, 16);

means to independently determine the location of each of said radiation emitter means (14, 16) on said first object (12) as a function of said radiation being transferred between at least two of said radiation emitter means (14, 16) on said first object (12) and said radiation sensor means (20, 22, 24) in fixed relation to said fixed coordination system (80);

means to determine the position and orientation of any point on said first object (12) in relation to said fixed coordinate system (80) by integrating the determined location of at least two of said radiation emitter means (14, 16) on said first object (12);

means to independently determine the location of each of said fiducial markers (71, 73, 75) on said second object in said fixed coordinate system (80);

means to determine the position and orientation of said second object (11) in said fixed coordinate system (80) by integrating the determined location of said fiducial markers (71, 73, 75) on said second object (11);

means to orient said previously taken three dimensional image data of said second object (11) such as to match the present time position and orientation of said second object (11) with the previously taken said image data;

means to integrate the present time determined position and orientation of said second object (11) with the present time determined position and orientation of said first object (12) in the same fixed coordinate system (80) whereby integrating the present time determined position and orientation of said first object (12) in relation to said previously taken three dimensional image data; and

means to, in present time, repeatedly determine the positions and orientations of said first object (12) with sufficient frequency to enable displaying the movement of said first object (12) in relation to said second object (11);

wherein, as a consequence of said repeated determinations and integrations of the positions and orientations of said first object (12), and correlation of said positions and orientations of said second object (11) with said previously taken three dimensional image data, an image representative of said first object (12) is correctly positioned in present time on said previously taken image data regardless of the movement of said first object (12) in relation to said fixed coordinate system (80).

2. A system as claimed in claim 1 wherein said first and second objects (11, 12) intersect, which system further comprises means to display so much of said previously taken three dimensional image data as corresponds to the intersecting location of said first and second objects (11, 12), and means to display said representation of said first object (12) on said image display in its correct present time position.

3. A system as claimed in claim 1 wherein the integration of the position and orientation of said second object (11) and said image data, the position and orientation of said first object (12), and the correlation of the position and orientation of said first object (12) with said image data are all determined substantially continuously.

4. The system as claimed in claim 1 wherein said previously taken three dimensional image data comprise a succession of substantially two dimensional slices through said second object (11).

5. The system as claimed in claim 1 wherein said radiation emitter means (14, 16) are disposed on said first object (12) and said sensor means (20, 22, 24) are spaced from said first and second objects (11) and are disposed in fixed relationship to said fixed coordinate system (80).

6. The system as claimed in claim 1 wherein said sensor means (20, 22, 24) are disposed on said first object (12) and said radiation emitter means (14, 16) are spaced from said first object (12) and are disposed in fixed relationship to said fixed coordinate system (80).

7. The system as claimed in claim 1 wherein said radiation is light.

8. The system as claimed in claim 7 wherein said radiation is visible light.

9. The system as claimed in claim 7 wherein light of different wave lengths is emitted from each different emitter means (14, 16).

10. The system as claimed in claim 7 wherein said light is pulse radiated from different of said radiation emitter means (14, 16) sequentially in a repeating order.

11. The system as claimed in claim 1 wherein said radiation emitter means (14, 16) and said sensor means (20, 22, 24) comprise means for detecting linear and rotational inertial motion.

12. The system as claimed in claim 1 wherein said previously taken image data are taken by a means which is not suitable for use during a surgical procedure.

**13.** The system as claimed in claim 1 wherein said first object is a surgical probe (12) and said second object is a surgical patient (11), wherein said surgical probe (12) is adapted to be partially inserted into said patient (11) such that said inserted portion is not visible to an inserter thereof.

**14.** The system as claimed in claim 13 wherein at least two emitter means (14, 16) are disposed on so much of said first object (12) as is not inserted into said patient (11), and wherein said so disposed emitter means (14, 16) on said first object (12) are a known distance from a tip of said probe which is adapted for insertion into said patient (11).

**15.** The system as claimed in claim 1 wherein said image data are selected from the group consisting of magnetic resonant image data, computed tomography data, ultra sound data, or X-radiation data.

**16.** The system as claimed in claim 1 wherein said second object is a patient (11), and said first object is an operating microscope, and wherein said image data are projected onto the field of said operating microscope.

**17.** The system as claimed in claim 1 wherein said second object (11) is a part of the anatomy of a human.

**18.** The system as claimed in claim 16 wherein the part of the anatomy is a head.

**19.** The system as claimed in claim 16 wherein there are reference points on said second object (11), which are distinguishing anatomical features, which are in fixed spacial relationship to said second object (11).

**20.** The system as claimed in claim 1 operated intermittently, but sufficiently frequently to depict the relative movement of said first object (12) relative to said second object (11).

**21.** The system as claimed in claim 2 including means for monitoring in present time the movement of said first object (12) in true relation to said previously, taken a three dimensional image data.

**22.** The system as claimed in claim 1 including means for selecting the slice of said previously taken image data which corresponds to the present time location of said first object (12) such that a representation of said first object (12) is correctly disposed on said image slice in present time.

**23.** The system as claimed in claim 1 including means to freeze a display of a previously taken image and a representation of said first object (12) for a finite period of time.

**24.** A method of controlling a computer system in an operation theatre for determining the present time location and orientation of a movable first object (12) with respect to a second object (11) and for graphically indicating the corresponding position and orientation of said first object (12) on a previously taken image of said second object (11), which comprises:

providing a present time three-dimensional fixed coordinate system (80);
providing at least three non-collinear fiducial markers (71, 73, 75) in fixed spatial relationship to said second object (11);
providing previously taken three-dimensional image data which geometrically describe said second object (11) including said fiducial markers (71, 73, 75);
at the present time, at sufficiently frequent intervals to follow present time movement, detecting the locations of at least two electromagnetic or acoustic radiation emitter means (14, 16) operatively associated with the first object (12) and in known spatial relationship to said first object (12);
at the present time, computing, from the detected positions of the electromagnetic or acoustic radiation emitter means (14, 16), at sufficiently frequent intervals to follow present time movement, the position and orientation of the first object (12) in present time relative to the fixed coordinate system (80);
determining the location of each of said fiducial markers (71, 73, 75) on said second object (11) in said fixed coordinate system (80);
determining the position and orientation of said second object (11) in said fixed coordinate system (80) by integrating the determined locations of said fiducial markers (71, 73, 75) on said second object (11);
orienting said previously taken three-dimensional image data of said second object (11) such as to match the present time position and orientation of said second object (11) with the previously taken image data;
integrating the present time determined position and orientation of said second object (11) with the present time determined position and orientation of said first object (12) whereby integrating the present time determined position and orientation of said first object (12) in relation to said previously taken three-dimensional image data; and
in present time, repeatedly determining the position and orientation of said first object (12) with sufficient frequency to enable displaying the movement of said first object (12) in relation to said second object (11);

wherein as a consequence of said repeated determination and integration of the position and orientation of said first object (12), and correlation of said position and orientation of said second object (11) with said previously taken three-dimensional image data, an image representative of said first object (12) is correctly positioned in present time on said previously taken image data regardless of the movement of said first object (12) in relation to said fixed coordinate system (80).

**Patentansprüche**

1. Medizinisches System (10) zur Bestimmung der Lage und Ausrichtung eines beweglichen ersten Objektes (12) in Ist-Zeit in Bezug auf ein zweites Objekt (11) und zur grafischen Anzeige der entsprechenden Stellung und Ausrichtung des ersten Objektes (12) auf einem zuvor aufgenommenen Bild des zweiten Objektes (11), das folgendes aufweist:

ein dreidimensionales, festes Ist-Zeit-Koordinatensystem (80);
mindestens drei elektromagnetische oder akustische Strahlungssensormittel (20, 22, 24) in fester räumlicher Beziehung zu dem dreidimensionalen, festen Ist-Zeit-Koordinatensystem (80), die von den Objekten (11, 12) beabstandet sind;
die ersten und zweiten Objekte (11, 12), die sich innerhalb des festen Koordinatensystems (80) befinden;
mindestens drei nicht kollineare Bezugsmarken (71, 73, 75) in fester räumlicher Beziehung zu dem zweiten Objekt (11) und bei bekannten Koordinaten in dem festen Koordinatensystem (80);
ein dreidimensionales lokales Koordinatensystem, welches in Bezug auf das zweite Objekt (11) fest ist;
zuvor aufgenommene dreidimensionale Bilddaten, welche das zweite Objekt (11) einschließlich der Bezugsmarken (71, 73, 75) geometrisch beschreiben;
mindestens zwei beabstandete elektromagnetische oder akustische Strahlungsemittermittel (14, 16), die auf dem ersten Objekt (12) angeordnet sind;
erste Bewegungsmittel, um das erste Objekt (12) relativ zu dem zweiten Objekt (11) und zu dem festen Koordinatensystem (80) zu bewegen;
Mittel zur Unterscheidung zwischen einer von einem beliebigen der Strahlungsemittermittel (14, 16) emittierten elektromagnetischen oder akustischen Strahlung und einer von allen anderen Strahlungsemittermitteln (14, 16) emittierten Strahlung;
Mittel zur unabhängigen Bestimmung der Lage jedes der Strahlungsemittermittel (14, 16) auf dem ersten Objekt (12) in Abhängigkeit von der zwischen mindestens zwei der Strahlungsemittermittel (14, 16) auf dem ersten Objekt (12) und dem Strahlungssensormittel (20, 22, 24), die sich in fester Beziehung zu diesem festen Koordinatensystem (80) befinden, übertragenen Strahlung
Mittel zur Bestimmung der Position und Ausrichtung jedes Punktes des ersten Objektes (12) in Bezug auf das feste Koordinatensystem (80) durch Integrieren der ermittelten Lage von mindestens zwei der Strahlungsemittermittel (14, 16) auf dem ersten Objekt (12);
Mittel zur unabhängigen Bestimmung der Lage jeder der Bezugsmarken (71, 73, 75) auf dem zweiten Objekt in dem festen Koordinatensystem (80);
Mittel zur Bestimmung der Position und Ausrichtung des zweiten Objektes (11) in dem festen Koordinatensystem (80) durch Integrieren der ermittelten Lage der Bezugsmarken (71, 73, 75) auf dem zweiten Objekt (11);
Mittel zur Ausrichtung der zuvor aufgenommenen dreidimensionalen Bilddaten des zweiten Objektes (11), um die Ist-Zeit-Position und die Ausrichtung des zweiten Objektes (11) an diese zuvor aufgenommenen Bilddaten anzupassen;
Mittel zur Integration der in Ist-Zeit bestimmten Position und Ausrichtung des zweiten Objektes (11) mit der in Ist-Zeit bestimmten Position und Ausrichtung des ersten Objektes (12) im selben festen Koordinatensystem (80), wodurch die in Ist-Zeit bestimmte Position und Ausrichtung des ersten Objektes (12) in Bezug auf die zuvor aufgenommenen dreidimensionalen Bilddaten integriert wird; und
Mittel zur wiederholten Bestimmung der Positionen und Ausrichtungen des ersten Objektes (12) in Ist-Zeit mit ausreichender Häufigkeit, um die Bewegung des ersten Objektes (12) in Bezug auf das zweite Objekt (11) anzeigen zu können;

wobei als eine Folge der wiederholten Bestimmungen und Integrationen der Positionen und Ausrichtungen des ersten Objektes (12) und der Korrelation der Positionen und Ausrichtungen des zweiten Objektes (11) mit den zuvor aufgenommenen dreidimensionalen Bilddaten ein für das erste Objekt (12) repräsentatives Bild in Ist-Zeit korrekt zu den zuvor aufgenommenen Bilddaten positioniert wird, ungeachtet der Bewegung des ersten Objektes (12) in Bezug auf das feste Koordinatensystem (80).

2. System nach Anspruch 1, **dadurch gekennzeichnet, dass** die ersten und zweiten Objekte (11, 12) sich schneiden, und das System weiter Mittel umfasst, um so viel der zuvor aufgenommenen dreidimensionalen Bilddaten, wie es der Schnittpunktlage dieses ersten und zweiten Objektes (11, 12) entspricht, anzuzeigen, und Mittel, um die Darstellung des ersten Objektes (12) auf dieser Bildanzeige in seiner korrekten Ist-Zeit-Position anzuzeigen.

3. System nach Anspruch 1, **dadurch gekennzeichnet, dass** die Integration der Position und Ausrichtung des zweiten Objektes (11) und der Bilddaten, die Position und Ausrichtung des ersten Objektes (12) und die Korrelation der Position und Ausrichtung des ersten Objektes (12) mit den Bilddaten sämtliche im Wesentlichen kontinuierlich bestimmt werden.

4. System nach Anspruch 1, **dadurch gekennzeichnet, dass** die zuvor aufgenommenen dreidimensionalen Bilddaten eine Aufeinanderfolge von im Wesentlichen zweidimensionalen Schichten durch das zweite Objekt (11) umfassen.

5. System nach Anspruch 1, **dadurch gekennzeichnet, dass** die Strahlungsemittermittel (14, 16) auf dem ersten Objekt (12) angeordnet sind und die Sensormittel (20, 22, 24) von den ersten und zweiten Objekten (11) beabstandet und in fester Beziehung zu dem festen Koordinatensystem (80) angeordnet sind.

6. System nach Anspruch 1, **dadurch gekennzeichnet, dass** die Sensormittel (20, 22, 24) auf dem ersten Objekt (12) angeordnet sind und die Strahlungsemittermittel (14, 16) von dem ersten Objekt (12) beabstandet und in fester Beziehung zu dem festen Koordinatensystem (80) angeordnet sind.

7. System nach Anspruch 1, **dadurch gekennzeichnet, dass** es sich bei der Strahlung um Licht handelt.

8. System nach Anspruch 7, **dadurch gekennzeichnet, dass** es sich bei der Strahlung um sichtbares Licht handelt.

9. System nach Anspruch 7, **dadurch gekennzeichnet, dass** Licht unterschiedlicher Wellenlänge von jedem der unterschiedlichen Emittermittel (14, 16) abgestrahlt wird.

10. System nach Anspruch 7, **dadurch gekennzeich-**net, dass das Licht von verschiedenen der Strahlungsemittermittel (14, 16), einer sich wiederholenden Reihenfolge folgend, gepulst abgestrahlt wird.

11. System nach Anspruch 1, **dadurch gekennzeichnet, dass** die Strahlungsemittermittel (14, 16) und die Sensormittel (20, 22, 24) Mittel für die Erfassung von linearer und drehender träger Bewegung umfassen.

12. System nach Anspruch 1, **dadurch gekennzeichnet, dass** die zuvor aufgenommenen Bilddaten durch Mittel, welche für die Verwendung während eines chirurgischen Eingriffs nicht geeignet sind, aufgenommen werden.

13. System nach Anspruch 1, **dadurch gekennzeichnet, dass** es sich bei dem ersten Objekt um eine chirurgische Sonde (12) und bei dem zweiten Objekt um einen chirurgischen Patienten (11) handelt, wobei die chirurgische Sonde (12) geeignet ist, um teilweise in den Patienten (11) derart eingeführt zu werden, dass der eingeführte Teil für dessen Einführenden nicht sichtbar ist.

14. System nach Anspruch 13, **dadurch gekennzeichnet, dass** mindestens zwei Emittermittel (14, 16) auf so viel von dem ersten Objekt (12) angeordnet sind, wie nicht in den Patienten (11) eingeführt wird, und wobei die so angeordneten Emittermittel (14, 16) auf dem ersten Objekt (12) sich in einer bekannten Entfernung von einer Spitze der Sonde befinden, welche für das Einführen in den Patienten (11) geeignet ist.

15. System nach Anspruch 1, **dadurch gekennzeichnet, dass** die Bilddaten aus einer Gruppe bestehend aus Magnetresonanzbilddaten, Computertomographiedaten, Ultraschalldaten oder Röntgenstrahlungsdaten ausgewählt sind.

16. System nach Anspruch 1, **dadurch gekennzeichnet, dass** es sich bei dem zweiten Objekt um einen Patienten (11) handelt und es sich bei dem ersten Objekt um ein Operationsmikroskop handelt und wobei die Bilddaten auf das Gesichtsfeld dieses Operationsmikroskops projiziert werden.

17. System nach Anspruch 1, **dadurch gekennzeichnet, dass** es sich bei dem zweiten Objekt (11) um einen Teil eines menschlichen Körpers handelt.

18. System nach Anspruch 16, **dadurch gekennzeichnet, dass** es sich bei dem Teil des Körpers um einen Kopf handelt.

19. System nach Anspruch 16, **dadurch gekennzeichnet, dass** sich auf dem zweiten Objekt (11) Referenzpunkte befinden, welche anatomische Merkmale, die sich in fester räumlicher Beziehung zu dem zweiten Objekt (11) befinden, unterscheiden.

20. System nach Anspruch 1, das mit Unterbrechungen arbeitet, jedoch ausreichend häufig, um die relative Bewegung des ersten Objektes (12) relativ zu dem zweiten Objekt (11) darstellen zu können.

21. System nach Anspruch 2, das Mittel für die Aufzeichnung der Bewegung des ersten Objektes (12) in richtiger Beziehung zu den zuvor aufgenommenen dreidimensionalen Bilddaten beinhaltet.

22. System nach Anspruch 1, das Mittel für die Auswahl derjenigen Schicht der zuvor aufgenommenen Bilddaten beinhaltet, welche der Ist-Zeit-Lage des ersten Objektes (12) entspricht, so dass eine Darstellung des ersten Objektes (12) auf der Bildschicht in Ist-Zeit korrekt angeordnet wird.

23. System nach Anspruch 1, das Mittel zum Einfrieren einer Anzeige eines zuvor aufgenommenen Bildes und einer Darstellung des ersten Objektes (12) für eine begrenzte Zeitspanne beinhaltet.

24. Verfahren zur Steuerung eines Computersystems in einem Operationssaal zur Bestimmung der Ist-Zeit-Lage und Ausrichtung eines beweglichen ersten Objektes (12) in Bezug auf ein zweites Objekt (11) und zur grafischen Anzeige der entsprechenden Position und Ausrichtung des ersten Objektes (12) auf einem zuvor aufgenommenen Bild des zweiten Objektes (11), das Folgendes aufweist:

Bereitstellen eines dreidimensionalen festen Ist-Zeit-Koordinatensystems (80);
Bereitstellen von mindestens drei nicht kollinearen Bezugsmarken (71, 73, 75) in fester räumlicher Beziehung zu dem zweiten Objekt (11);
Bereitstellen von zu einem früheren Zeitpunkt aufgenommenen und das zweite Objekt (11) einschließlich der Bezugsmarken (71, 73, 75) geometrisch beschreibenden, dreidimensionalen Bilddaten;
Erfassen der Lagen von mindestens zwei elektromagnetischen oder akustischen Strahlungsemittermitteln (14, 16), die dem ersten Objekt (12) funktional zugeordnet sind und in bekannter räumlicher Beziehung zu dem ersten Objekt (12) stehen, in Ist-Zeit in ausreichend häufigen Intervallen, um die Bewegung in Ist-Zeit verfolgen zu können;
Berechnen in Ist-Zeit aus den erfassten Positionen der elektromagnetischen oder akustischen Strahlungsemittermittel (14,16) in ausreichend häufigen Intervallen, um der Ist-Zeit-Bewegung in Ist-Zeit folgen zu können, der Position und der Ausrichtung des ersten Objektes (12) in Bezug auf das feste Koordinatensystem (80);
Bestimmen der Lage jeder der Bezugsmarken (71, 73, 75) auf dem zweiten Objekt (11) in dem festen Koordinatensystem (80);
Bestimmen der Position und Ausrichtung des zweiten Objektes (11) in dem festen Koordinatensystem (80) durch Integration der ermittelten Lagen der Bezugsmarken (71, 73, 75) auf dem zweiten Objekt (11);
Ausrichten der zuvor aufgenommenen dreidimensionalen Bilddaten des zweiten Objektes (11), um die Position und Ausrichtung des zweiten Objektes (11) an die zuvor aufgenommenen Bilddaten anzupassen;
Integration der in Ist-Zeit bestimmten Position und Ausrichtung des zweiten Objektes (11) mit der in Ist-Zeit bestimmten Position und Ausrichtung des ersten Objektes (12), wodurch die in Ist-Zeit bestimmte Position und Ausrichtung des ersten Objektes (12) in Bezug auf die zuvor aufgezeichneten dreidimensionalen Bilddaten integriert wird; und
wiederholtes Bestimmen der Position und Ausrichtung des ersten Objektes (12) in Ist-Zeit mit ausreichender Häufigkeit, um ein Anzeigen der Bewegung des ersten Objektes (12) in Bezug auf das zweite Objekt (11) zu ermöglichen;

wobei als eine Konsequenz der wiederholten Bestimmung und Integration der Position und Ausrichtung des ersten Objektes (12) und der Korrelation der Position und Ausrichtung des zweiten Objektes (11) mit den zuvor aufgenommenen dreidimensionalen Bilddaten ein für das erste Objekt (12) repräsentatives Bild auf den zuvor aufgenommenen Bilddaten in Ist-Zeit korrekt positioniert wird, ungeachtet der Bewegung des ersten Objektes (12) in Bezug auf das feste Koordinatensystem (80).

## Revendications

1. Système médical (10) de détermination de l'emplacement et de l'orientation à l'instant présent d'un premier objet mobile (12) par rapport à un second objet (11) et d'indication graphique de la position et de l'orientation correspondantes dudit premier objet (12) sur une image précédemment prise dudit second objet (11), comprenant :

un repère fixe tridimensionnel à l'instant présent (80) ;
au moins trois moyens de détection de rayonnement électromagnétique ou acoustique (20, 22, 24) dans une relation spatiale fixe audit repère fixe tridimensionnel à l'instant présent (80) qui sont espacés desdits objets (11, 12) ; lesdits premier et second objets (11, 12) situés dans ledit repère fixe (80) ;

au moins trois balises de repère non colinéaires (71, 73, 75) dans une relation spatiale fixe audit second objet (11) et avec des coordonnées connues dans ledit repère fixe (80) ;

un repère local tridimensionnel qui est fixe relativement audit second objet (11) ;

des données-image tridimensionnelles précédemment acquises qui décrivent géométriquement ledit second objet (11) y compris lesdites balises de repère (71, 73, 75) ;

au moins deux moyens d'émission de rayonnement électromagnétique ou acoustique (14, 16) espacés l'un de l'autre disposés sur ledit premier objet (12) ;

des premiers moyens de mouvement pour mouvoir ledit premier objet (12) par rapport audit second objet (11) et audit repère fixe (80) ;

des moyens pour distinguer un rayonnement électromagnétique ou acoustique émis à partir d'un quelconque moyen d'émission de rayonnement (14, 16) d'un rayonnement émis depuis tous les autres moyens d'émission de rayonnement (14, 16);

des moyens pour déterminer de manière indépendante l'emplacement de chacun desdits moyens d'émission de rayonnement (14, 16) sur ledit premier objet (12) en fonction dudit rayonnement transmis entre au moins deux desdits moyens d'émission de rayonnement (14, 16) sur ledit premier objet (12) et lesdits moyens de détection de rayonnement (20, 22, 24) dans une relation fixe audit repère fixe (80) ;

des moyens pour déterminer la position et l'orientation de tout point sur ledit premier objet (12) relativement audit repère fixe (80) en intégrant l'emplacement déterminé d'au moins deux desdits moyens d'émission de rayonnement (14, 16) sur ledit premier objet (12);

des moyens pour déterminer de manière indépendante l'emplacement de chacune desdites balises de repère (71, 73, 75) sur ledit second objet dans ledit repère fixe (80) ;

des moyens pour déterminer la position et l'orientation dudit second objet (11) dans ledit repère fixe (80) en intégrant l'emplacement déterminé desdites balises de repère (71, 73, 75) sur ledit second objet (11) ;

des moyens pour orienter lesdites données-image tridimensionnelles précédemment acquises dudit second objet (11) de manière à corréler la position et l'orientation dudit second objet (11) à l'instant présent avec lesdites données-image précédemment acquises ;

des moyens pour intégrer la position et l'orientation déterminées dudit second objet (11) à l'instant présent avec la position et l'orientation déterminées dudit premier objet (12) à l'instant présent dans le même repère fixe (80) en intégrant ainsi la position et l'orientation déterminées dudit premier objet (12) à l'instant présent relativement auxdites données-image tridimensionnelles précédemment acquises ; et

des moyens pour, à l'instant présent, déterminer de manière répétée les positions et les orientations dudit premier objet (12) avec une fréquence suffisante pour permettre l'affichage du mouvement dudit premier objet (12) relativement audit second objet (11);

dans lequel, en conséquence desdites déterminations et intégrations répétées des positions et des orientations dudit premier objet (12), et de la corrélation desdites positions et orientations dudit second objet (11) avec lesdites données-image tridimensionnelles précédemment acquises, une image représentative dudit premier objet (12) est correctement positionnée à l'instant présent sur lesdites données-image précédemment acquises indifféremment du mouvement dudit premier objet (12) relativement audit repère fixe (80).

2. Système selon la revendication 1 dans lequel lesdits premier et second objets (11, 12) s'entrecroisent, lequel système comprend en outre des moyens pour afficher la partie desdites données-image tridimensionnelles précédemment acquises qui correspond à l'emplacement de l'intersection desdits premier et second objets (11, 12), et des moyens pour afficher ladite représentation dudit premier objet (12) sur ledit affichage d'image dans sa position à l'instant présent correcte.

3. Système selon la revendication 1 dans lequel l'intégration de la position et de l'orientation dudit second objet (11) et desdites données-image, la position et l'orientation dudit premier objet (12), et la corrélation de la position et de l'orientation dudit premier objet (12) avec lesdites données-image sont toutes déterminées de manière substantiellement continue.

4. Système selon la revendication 1 dans lequel lesdites données-image tridimensionnelles précédemment acquises comprennent une succession de tranches substantiellement bidimensionnelles à travers ledit second objet (11).

5. Système selon la revendication 1 dans lequel lesdits moyens d'émission de rayonnement (14, 16) sont disposés sur ledit premier objet (12) et lesdits moyens de détection (20, 22, 24) sont espacés desdits premier et second objets (11, 12) et sont disposés dans une relation fixe audit repère fixe (80).

6. Système selon la revendication 1 dans lequel lesdits moyens de détection (20, 22, 24) sont disposés sur ledit premier objet (12) et lesdits moyens d'émission de rayonnement (14, 16) sont espacés dudit premier objet (12) et sont disposés dans une relation fixe audit repère fixe (80).

7. Système selon la revendication 1 dans lequel ledit rayonnement est la lumière.

8. Système selon la revendication 7 dans lequel ledit rayonnement est la lumière visible.

9. Système selon la revendication 7 dans lequel une lumière de différentes longueurs d'onde est émise depuis chaque moyen d'émission différent (14, 16).

10. Système selon la revendication 7 dans lequel ladite lumière est rayonnée par impulsion à partir de l'un différent desdits moyens d'émission de rayonnement (14, 16) de manière séquentielle dans un ordre se répétant.

11. Système selon la revendication 1 dans lequel lesdits moyens d'émission de rayonnement (14, 16) et lesdits moyens de détection (20, 22, 24) comprennent des moyens pour détecter un mouvement d'inertie linéaire et rotationnel.

12. Système selon la revendication 1 dans lequel lesdites données-image précédemment acquises sont acquises par un moyen qui n'est pas adapté à une utilisation au cours d'une procédure chirurgicale.

13. Système selon la revendication 1 dans lequel ledit premier objet est une sonde chirurgicale (12) et ledit second objet est un patient chirurgical (11), dans lequel ladite sonde chirurgicale (12) est adaptée pour être partiellement insérée dans ledit patient (11) de telle sorte que ladite partie insérée n'est pas visible de son introducteur.

14. Système selon la revendication 13 dans lequel au moins deux moyens d'émission (14, 16) sont disposés sur la partie dudit premier objet (12) qui n'est pas insérée dans ledit patient (11), et dans lequel lesdits moyens d'émission (14, 16) ainsi disposés sur ledit premier objet (12) sont à une distance connue d'une pointe de ladite sonde qui est adaptée à une insertion dans ledit patient (11).

15. Système selon la revendication 1 dans lequel lesdites données-image sont choisies dans le groupe consistant en données-image de résonance magnétique, données de tomographie par ordinateur, données d'ultrasons, ou données de rayons X.

16. Système selon la revendication 1 dans lequel ledit second objet est un patient (11), et ledit premier objet est un microscope opératoire, et dans lequel lesdites données-image sont projetées sur le champ dudit microscope opératoire.

17. Système selon la revendication 1 dans lequel ledit second objet (11) est une partie de l'anatomie d'un homme.

18. Système selon la revendication 17 dans lequel la partie de l'anatomie est une tête.

19. Système selon la revendication 16 dans lequel des points de référence se trouvent sur ledit second objet (11), lesquels sont des caractéristiques anatomiques distinctives, qui sont dans une relation spatiale fixe audit second objet (11).

20. Système selon la revendication 1 fonctionnant par intermittence, mais suffisamment fréquemment pour représenter le mouvement relatif dudit premier objet (12) par rapport audit second objet (11).

21. Système selon la revendication 2 comportant des moyens pour surveiller à l'instant présent le mouvement dudit premier objet (12) dans une relation vraie auxdites données-image tridimensionnelles précédemment acquises.

22. Système selon la revendication 1 comportant des moyens pour sélectionner la tranche desdites données-image précédemment acquises qui correspond à l'emplacement dudit premier objet (12) à l'instant présent de telle sorte qu'une représentation dudit premier objet (12) est correctement disposée sur ladite tranche d'image à l'instant présent.

23. Système selon la revendication 1 comportant des moyens pour figer l'affichage d'une image précédemment prise et une représentation dudit premier objet (12) sur une période de temps finie.

24. Méthode de contrôle d'un système informatique dans une salle d'opération pour déterminer l'emplacement et l'orientation à l'instant présent d'un premier objet mobile (12) par rapport à un second objet (11) et pour indiquer graphiquement la position et l'orientation correspondantes dudit premier objet (12) sur une image précédemment prise dudit second objet (11), comprenant :

la fourniture d'un repère fixe tridimensionnel à l'instant présent (80) ;
la fourniture d'au moins trois balises de repère non colinéaires (71, 73, 75) dans une relation spatiale fixe audit second objet (11);
la fourniture de données-image tridimensionnelles précédemment acquises qui décrivent géométriquement ledit second objet (11) y compris lesdites balises de repère (71, 73, 75);

la détection à l'instant présent, à des intervalles suffisamment fréquents pour suivre un mouvement à l'instant présent, des emplacements d'au moins deux moyens d'émission de rayonnement électromagnétique ou acoustique (14, 16) fonctionnellement associés au premier objet (12) et dans une relation spatiale connue audit premier objet (12) ;

le calcul à l'instant présent, à partir des positions détectées des moyens d'émission de rayonnement électromagnétique ou acoustique (14, 16), à des intervalles suffisamment fréquents pour suivre un mouvement à l'instant présent, de la position et de l'orientation du premier objet (12) à l'instant présent par rapport au repère fixe (80) ;

la détermination de l'emplacement de chacune desdites balises de repère (71, 73, 75) sur ledit second objet (11) dans ledit repère fixe (80) ;

la détermination de la position et de l'orientation dudit second objet (11) dans ledit repère fixe (80) par l'intégration des emplacements déterminés desdites balises de repère (71, 73, 75) sur ledit second objet (11);

l'orientation desdites données-image tridimensionnelles précédemment acquises dudit second objet (11) de manière à corréler la position et l'orientation dudit second objet (11) à l'instant présent avec les données-image précédemment acquises ;

l'intégration de la position et de l'orientation déterminées dudit second objet (11) à l'instant présent avec la position et l'orientation déterminées dudit premier objet (12) à l'instant présent en intégrant ainsi la position et

l'orientation déterminées dudit premier objet (12) à l'instant présent relativement auxdites données-image tridimensionnelles précédemment acquises ; et

la détermination à l'instant présent, de manière répétée, de la position et de l'orientation dudit premier objet (12) avec une fréquence suffisante pour permettre l'affichage du mouvement dudit premier objet (12) relativement audit second objet (11);

dans laquelle, en conséquence desdites déterminations et intégrations répétées de la position et de l'orientation dudit premier objet (12), et de la corrélation desdites positions et orientations dudit second objet (11) avec lesdites données-image tridimensionnelles précédemment acquises, une image représentative dudit premier objet (12) est correctement positionnée à l'instant présent sur lesdites données-image précédemment acquises indifféremment du mouvement dudit premier objet (12) relativement audit repère fixe (80).

Fig. 1A

Fig. 1B

Fig. 2

Fig. 3

Fig. 4

Fig. 5

EP 1 219 259 B1

Fig. 6

PROBE
DIMENSIONS

*21*

I-DIMENSIONAL
MEASUREMENTS
FROM
SENSORS

```
┌─────────────────┐        ┌─────────────────┐
│ COMPUTE 3-D     │        │ COMPUTE PROBE   │  39
│ PROBE EMITTER   │───────▶│ TIP COORDINATES │
│ COORDINATES     │        │ (AND PROBE DIR- │
│                 │        │ ECTION) IN THE  │
└─────────────────┘        │ SENSOR SPACE    │
                           └─────────────────┘
```

45

REFERENCE POINT
COORDINATES
IN IMAGE
SPACE

```
┌─────────────────┐        ┌─────────────────┐
│ GENERATE THE    │        │ TRANSFORM PROBE │  44
│ TRANSFORMATION  │        │ COORDINATES TO  │
│ BETWEEN IMAGE   │───────▶│ MODEL SPACE     │
│ AND SENSOR      │        │                 │
│ COORDINATE      │        └─────────────────┘
│ SPACES          │
└─────────────────┘
```

46

```
┌───────────┐      ┌─────────────────┐
3-D IMAGE    │ RANDOM-   │      │ COMPUTE PLANE   │
MODEL ───────│ ACCESS    │◀─────│ OF 2-D SLICE    │
DATA         │ MEMORY    │─────▶│ THROUGH THE     │
             └───────────┘      │ 3-D IMAGE MODEL │
                                │ AT PROBE TIP    │
                                └─────────────────┘
```

41                    43

VIEWING
PARAMETERS

```
                                ┌─────────────────┐
                                │ TRANSFORM SLICE │
                                │ TO SCREEN       │
                                │ COORDINATES;    │
                                │ SHOW CURSOR     │
                                │ AT PROBE TIP    │
                                └─────────────────┘
```

40          48        47

# Fig. 7

```
                                ┌─────────────────┐
                                │ DISPLAY THE     │
                                │ SLICE           │
                                │ GRAPHICALLY     │
                                └─────────────────┘
```

Fig. 8